(19) **Europäisches Patentamt / European Patent Office / Office européen des brevets**

(11) **EP 4 745 986 A2**

(12) # EUROPEAN PATENT APPLICATION

(43) Date of publication:
20.05.2026 Bulletin 2026/21

(21) Application number: 25216758.0

(22) Date of filing: 18.11.2025

(51) International Patent Classification (IPC):
*G16H 40/63* (2018.01)     *G16H 20/40* (2018.01)

(52) Cooperative Patent Classification (CPC):
**G16H 40/63; G16H 20/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 18.11.2024 US 202418951268

(71) Applicant: **Cilag GmbH International**
**6300 Zug (CH)**

(72) Inventors:
- **LEUCK, Stephen M.**
  **Cincinnati, 45242 (US)**
- **DENZINGER, Kristen G.**
  **Cincinnati, 45242 (US)**
- **WRIGHT, Lindsay**
  **Cincinnati, 45242 (US)**
- **SCHLEY, Nicholas D.**
  **Cincinnati, 45242 (US)**
- **MESSERLY, Jeffrey D.**
  **Cincinnati, 45242 (US)**

(74) Representative: **Carpmaels & Ransford LLP**
**One Southampton Row**
**London WC1B 5HA (GB)**

(54) **INTELLIGENT INSUFFLATION AND SMOKE EVACUATION**

(57) A surgical system is disclosed including an evacuation module and a controller. The evacuation module comprises a motor and a pump drivable by the motor to draw smoke from a patient. The controller is operable to receive a first input indicative of a first step of a surgical procedure, set a first motor speed of the motor based on the first input, receive a second input indicative of a second step of the surgical procedure, and adjust the first motor speed to a second motor speed different than the first motor speed based on the second input.

EP 4 745 986 A2

## Description

## BACKGROUND

The present disclosure relates to surgical systems and, more particularly, insufflation and smoke evacuation systems used in surgical systems.

During a surgical procedure involving an energy device, insufflators are often used to provide insufflation to a patient's body cavity and thereby enhance visibility and access to the same. During the surgical procedure, smoke can be generated at a surgical site as the energy device is utilized. Surgical smoke evacuators are configured to evacuate smoke, as well as fluid and/or particulate, from the surgical site.

## BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.

FIG. 1 is a block diagram of a computer-implemented interactive surgical system, in accordance with at least one aspect of the present disclosure.

FIG. 2 is a diagram of various modules, including an energy module, an evacuation module, and an insufflation module, and other components that are combinable to customize modular surgical systems, in accordance with at least one aspect of the present disclosure.

FIG. 3 is the energy module of FIG. 2 and various surgical instruments usable therewith, in accordance with at least one aspect of the present disclosure.

FIG. 4A is a first illustrative modular surgical system configuration including a header module and a display screen that renders a graphical user interface (GUI) for relaying information regarding modules connected to the header module, in accordance with at least one aspect of the present disclosure.

FIG. 4B is an isometric view of the modular surgical system shown in FIG. 4A mounted to a cart, in accordance with at least one aspect of the present disclosure.

FIG. 5 is a second illustrative modular surgical system configuration including a header module, a display screen, two energy modules, and an evacuation module connected together and mounted to a cart, in accordance with at least one aspect of the present disclosure.

FIG. 6 is a block diagram of an example modular surgical system, in accordance with at least one aspect of the present disclosure.

FIG. 7 is a schematic depicting internal components of the insufflation module of FIG. 2, in accordance with at least one aspect of the present disclosure.

FIG. 8 is a schematic depicting internal components within the evacuation module of FIG. 2, in accordance with at least one aspect of the present disclosure.

FIG. 9 is a block diagram of a modular surgical system including a display screen operable to display interactable widgets, in accordance with at least one aspect of the present disclosure.

FIG. 10 is a method of controlling the modular surgical system of FIG. 9, in accordance with at least one aspect of the present disclosure.

FIG. 11 is a method of controlling the modular surgical system of FIG. 9, in accordance with at least one aspect of the present disclosure.

FIG. 12 is a method of controlling the modular surgical system of FIG. 9, in accordance with at least one aspect of the present disclosure.

FIG. 13 is a block diagram of a modular surgical system including a display screen operable to display a final evacuation widget, in accordance with at least one aspect of the present disclosure.

FIG. 14 is a method of controlling the modular surgical system of FIG. 13, in accordance with at least one aspect of the present disclosure.

FIG. 15 is a method of controlling the modular surgical system of FIG. 13, in accordance with at least one aspect of the present disclosure.

## DETAILED DESCRIPTION

Applicant of the present application owns the following U.S. Patent Applications filed concurrently herewith, the disclosure of each of which is hereby incorporated by reference in their entirety herein:

U.S. Patent Application No. 18/950,801, titled IMPROVED FILTER LIFE IN SURGICAL smoke EVACUATION SYSTEMS;

U.S. Patent Application No. 18/950,899, titled AIR MANAGEMENT IN SURGICAL SMOKE EVACUATION SYSTEMS; and

U.S. Patent Application No. 18/951,342, titled SETTING EVACUATION MOTOR SPEEDS FOR SURGICAL TOOL EVACUATION MODULES.

The present disclosure relates to energy devices, insufflation systems for providing insufflation gas to a patient, and evacuation systems for evacuating smoke and/or other fluids and/or particulates from a surgical site.

Smoke is often generated during a surgical procedure that utilizes one or more energy devices supplied with energy (power) from a generator. Energy devices use energy to affect (treat) tissue, and include devices with tissue-contacting electrodes, such as an electrosurgical device having one or more radio frequency (RF) electrodes, and devices with vibrating surfaces, such as an ultrasonic device having an ultrasonic blade. For an electrosurgical device, a generator is configured to generate oscillating electric currents to energize the electrodes. For an ultrasonic device, a generator is configured to generate ultrasonic vibrations to energize the ultrasonic blade. Generators are further described herein. An evacuation module is utilized to control (evacuate) an amount of smoke generated by the energy devices during use thereof.

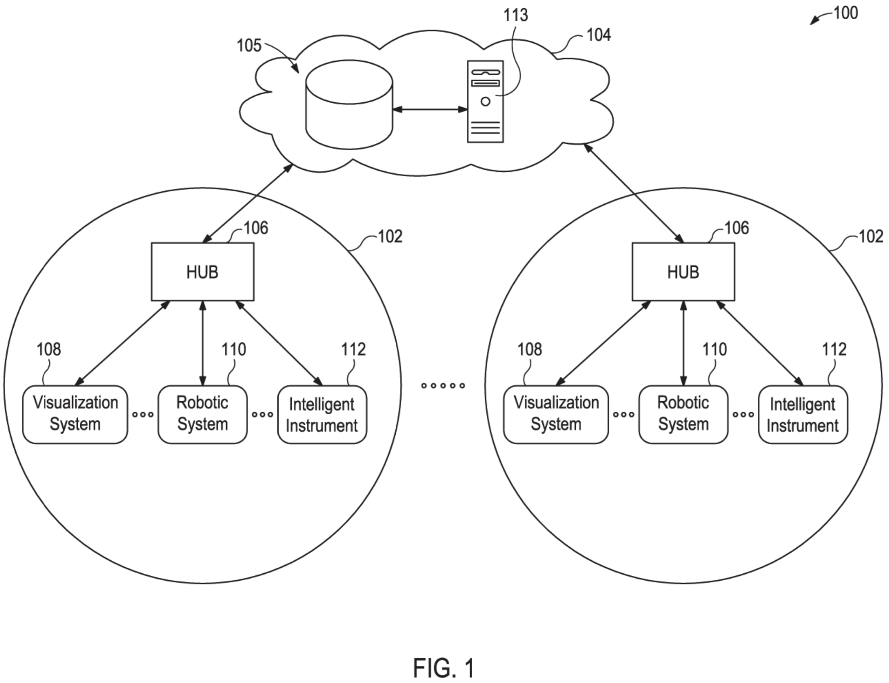

FIG. 1 is a block diagram of a computer-implemented interactive surgical system 100 (hereinafter "the surgical system 100") that may be used in accordance with at least one aspect of the present disclosure. The surgical system 100 includes one or more sub-surgical systems 102 and a cloud-based system (e.g., the cloud 104) that may include a remote server 113 in communication with a storage device 105. Each sub-surgical system 102 includes at least one surgical hub 106 in communication with the cloud 104 that may include a remote server 113.

In one example, as illustrated in FIG. 1, each sub-surgical system 102 includes a visualization system 108, a robotic system 110, and a handheld intelligent surgical instrument 112, which are configured to communicate with one another and/or the hub 106. In some aspects, each sub-surgical system 102 may include an M number of hubs 106, an N number of visualization systems 108, an O number of robotic systems 110, and a P number of handheld intelligent surgical instruments 112, where M, N, O, and P are integers greater than or equal to one. The surgical system 100 is described in more detail in U.S. Patent No. 11,666,368, entitled "METHOD FOR CONSTRUCTING AND USING A MODULAR SURGICAL ENERGY SYSTEM WITH MULTIPLE DEVICES", issued June 6, 2023, and is hereby incorporated by reference in its entirety herein.

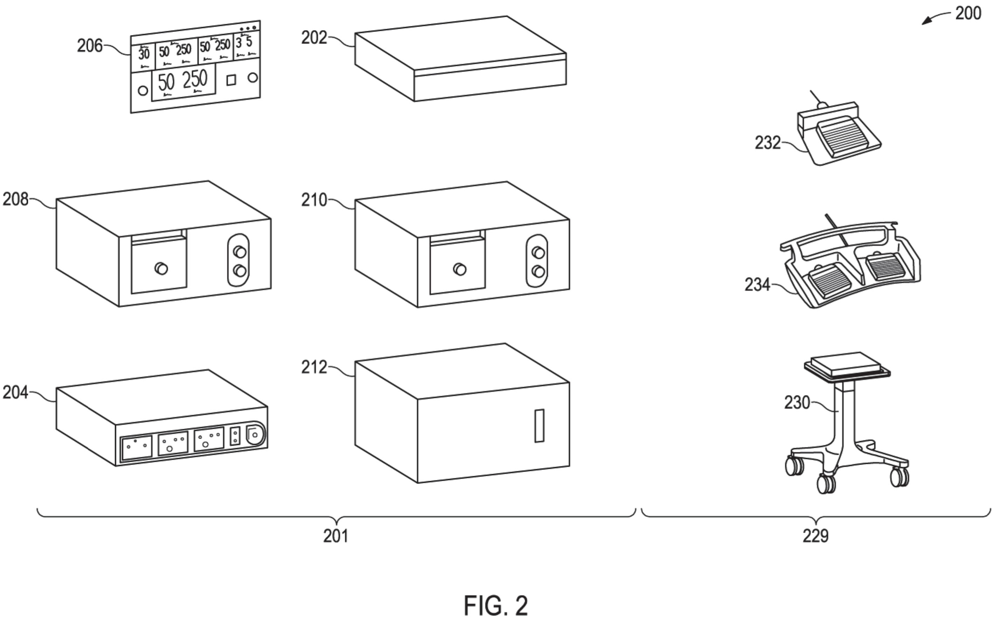

Referring now to FIG. 2, an example surgical hub 106 (FIG. 1) can be embodied as a modular surgical system 200 that can include a variety of different modules 201 that are connectable together in a stacked configuration. In one aspect, the modules 201 can be both physically and communicably coupled together when stacked or otherwise connected together into a singular assembly. Further, the modules 201 can be interchangeably connectable together in different combinations or arrangements. In one aspect, each of the modules 201 can include a consistent or universal array of connectors disposed along their upper and lower surfaces, thereby allowing any module 201 to be connected to another module 201 in any arrangement (except that, in some aspects, a particular module type, such as the header module 202, can be configured to serve as the uppermost module within the stack, for example). In an alternative aspect, the modular surgical system 200 can include a housing that is configured to receive and retain the modules 201. The modular surgical system 200 can also include a variety of different components or accessories that are also connectable to or otherwise associatable with the modules 201.

The modular surgical system 200 can be assembled from a variety of different modules 201, some examples of which are illustrated in FIG. 2. Each of the different types of modules 201 can provide different functionality, thereby allowing the modular surgical system 200 to be assembled into different configurations to customize the functions and capabilities of the modular surgical system 200 (e.g., by customizing the modules 201 that are included in each modular surgical system 200). The modules 201 of the modular surgical system 200 can include, for example, a header module 202 (which can include a display screen 206), an energy module 204, an evacuation module 208, an insufflation module 210, and a visualization module 212.

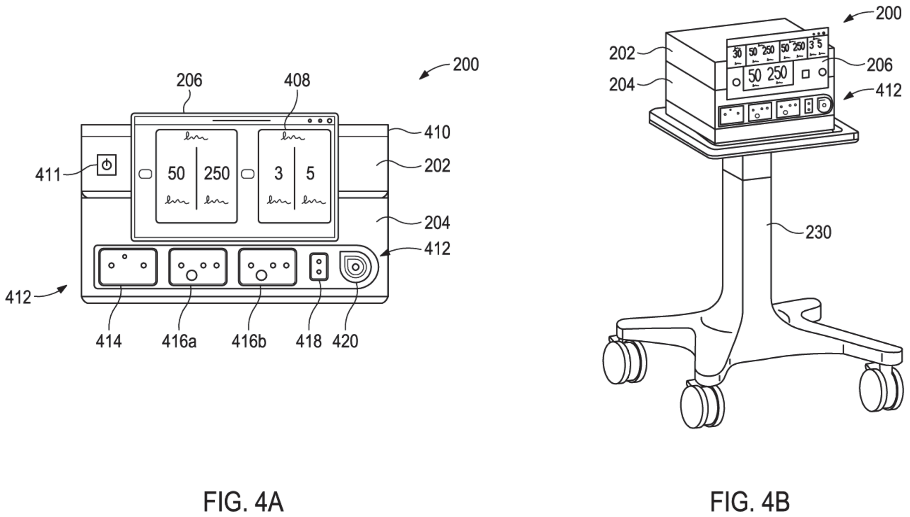

In the depicted aspect, the header module 202 is configured to serve as the top or uppermost module within the modular surgical system stack and can thus lack connectors along its top surface. In another aspect, the header module 202 can be configured to be positioned at the bottom or the lowermost module within the modular surgical system stack (*i.e.,* a "footer" module) and can thus lack connectors along its bottom surface. In yet another aspect, the header module 202 can be configured to be positioned at an intermediate position within the modular surgical system stack and can thus include connectors along both its bottom and top surfaces. The header module 202 can be configured to control the system-wide settings of each module 201 and component connected thereto through physical controls 411 (FIG. 4A) thereon and/or a graphical user interface (GUI) 408 (FIG. 4A) rendered on the display screen 206. Such settings could include the activation of the modular surgical system 200, the volume of alerts, the footswitch settings, the settings icons, the appearance or configuration of the user interface, the surgeon profile logged into the modular surgical system 200, and/or the type of surgical procedure being performed. The header module 202 can also be configured to provide communications, processing, and/or power for the modules 201 that are connected to the header module 202.

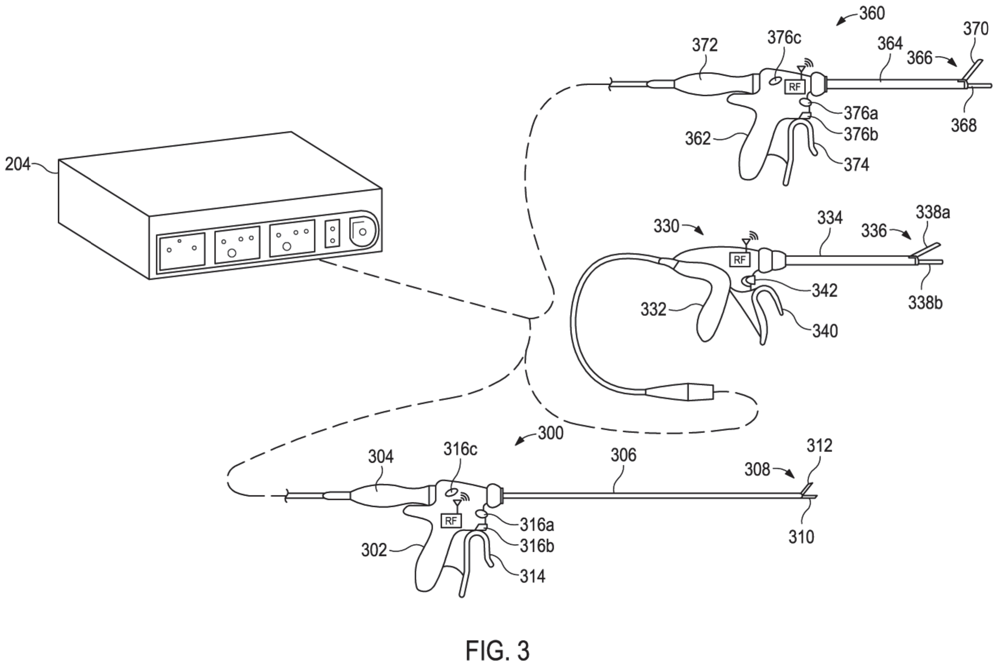

The energy module 204, alternately referred to as a generator module, can be configured to generate one or multiple energy modalities for driving electrosurgical and/or ultrasonic surgical instruments connected thereto. For example, referring to FIG. 3, the generator 204 is configured to drive multiple surgical instruments 300, 330, 360. The first surgical instrument is an ultrasonic surgical instrument 300 and comprises a handpiece 302 (HP), an ultrasonic transducer 304, a shaft 306, and an end effector 308. The end effector 308 comprises an ultrasonic blade 310 acoustically coupled to the ultrasonic transducer 304 and a clamp arm 312. The handpiece 302 comprises a trigger 314 to operate the clamp arm 312 and a combination of toggle buttons 316a, 316b, 316c to energize and drive the ultrasonic blade 310 or other function. The toggle buttons 316a-c can be configured to energize the ultrasonic transducer 304 with the generator 204.

The generator 204 is also configured to drive the second surgical instrument 330, which is an RF electrosurgical instrument and comprises a handpiece 332 (HP), a shaft 334, and an end effector 336. The end effector 336 comprises electrodes in clamp arms 338a, 338b and return through an electrical conductor portion of the shaft 334. The electrodes are coupled to and energized by a bipolar energy source within the generator 204. The handpiece 332 includes a trigger 340 manually actuatable to operate the clamp arms 338a,b, and an energy button 342 to actuate an energy switch to energize the electrodes in the end effector 336.

The generator 204 is also configured to drive the third surgical instrument 360, which is a multifunction surgical instrument 360 and comprises a handpiece 362 (HP), a shaft 364, and an end effector 366. The end effector 366 comprises an ultrasonic blade 368 and a clamp arm 370. The ultrasonic blade 368 is acoustically coupled to an ultrasonic transducer 372. The handpiece 362 includes a trigger 374 to operate the clamp arm 370, and a combination of toggle buttons 376a, 376b, 376c to energize and drive the ultrasonic blade 368 or other function. The toggle buttons 376a-c can be configured to energize the ultrasonic transducer 372 with the generator 204 and energize the ultrasonic blade 368 with a bipolar energy source also contained within the generator 204. Further aspects of the surgical instruments are described in U.S. Patent No. 10,624,691, entitled "TECHNIQUES FOR OPERATING GENERATOR FOR DIGITALLY GENERATING ELECTRICAL SIGNAL WAVEFORMS AND SURGICAL INSTRUMENTS", issued April 21, 2020, which is herein incorporated by reference in its entirety herein.

The evacuation module 208 (FIG. 2) can be configured to evacuate smoke, fluid, and/or particulates generated by the application of therapeutic energy to the tissue by one or more of the surgical instruments 300, 330, 360. Example evacuation modules are described in more detail elsewhere herein, as well as in U.S. Patent No. 11,602,393, entitled "SURGICAL EVACUATION SENSING AND GENERATOR CONTROL", issued March 14, 2023, which is hereby incorporated by reference in its entirety herein. The insufflation module 210 (FIG. 2) can be configured to blow air or gas into a body cavity of a patient to inflate it for diagnostic or surgical procedures, thereby providing better visibility and access during procedures.

The visualization module 212 (FIG. 2) can be configured to interface with visualization devices (i.e., scopes) and accordingly provide increased visualization capabilities. Example visualization modules and systems are described in more detail in U.S. Patent No. 11,284,963, entitled "METHOD OF USING IMAGING DEVICES IN SURGERY", issued March 29, 2022, which is hereby incorporated by reference in its entirety herein.

Referring again to FIG. 2, the modular surgical system 200 can further include a variety of accessories 229 that are connectable to the modules 201 for controlling the functions thereof or that are otherwise configured to work in conjunction with the modular surgical system 200. The accessories 229 can include, for example, a single-pedal footswitch 232, a dual-pedal footswitch 234, and a cart 230 for supporting the modular surgical system 200 thereon. The footswitches 232, 234 can be configured to control the activation or function of particular energy modalities output by the energy module 204, for example.

By utilizing modular components, the depicted modular surgical system 200 provides a surgical platform that grows with the availability of technology and is customizable to the needs of the facility and/or surgeons. Further, the modular surgical system 200 supports combo devices (e.g., dual electrosurgical and ultrasonic energy generators) and supports software-driven algorithms for customized tissue effects. Still further, the surgical system architecture reduces the capital footprint by combining multiple technologies critical for surgery into a single system.

The various modular components utilizable in connection with the modular surgical system 200 can include monopolar energy generators, bipolar energy generators, dual electrosurgical/ultrasonic energy generators, display screens, and various other modules and/or other components described elsewhere herein.

Referring now to FIG. 4A, the header module 202 can, in some aspects, include the display screen 206 that renders a GUI 408 for relaying information regarding the modules 201 (FIG. 2) connected to the header module 202. In some aspects, the GUI 408 of the display screen 206 can provide a consolidated point of control of all of the modules 201 making up the particular configuration of the modular surgical system 200. In alternative aspects, the header module 202 can lack the display screen 206, or the display screen 206 can be detachably connected (removably attachable) to a housing 410 of the header module 202. In such aspects, the header module 202 can be communicably couplable to an external system that is configured to display the information generated by the modules 201 of the modular surgical system 200. For example, in robotic surgical applications, the modular surgical system 200 can be communicably couplable to a robotic cart or robotic control console, which is configured to display the information generated by the modular surgical system 200 to the operator of the robotic surgical system. As another example, the modular surgical system 200 can be communicably couplable to a mobile display that can be carried or secured to a surgical staff member for viewing thereby. In aspects utilizing a user interface that is separate from or otherwise distinct from the modular surgical system 200, the user interface can be wirelessly connectable with the modular surgical system 200 as a whole or one or more modules 201 thereof such that the user interface can display information from the connected modules 200 thereon.

Referring still to FIG. 4A, the energy module 204 can include a port assembly 412 including (providing) a number of different ports configured to deliver different energy modalities to corresponding surgical instruments (e.g., surgical instruments 300, 330, 360 of FIG. 3, for example) that are connectable thereto. In the particular aspect illustrated in FIGS. 4A, the port assembly 412 includes a bipolar port 414, a first monopolar port 416a, a second monopolar port 416b, a neutral electrode port 418 (to which a monopolar return pad is connectable), and a combination energy port 420. However, this particular combination of ports is simply provided for illustrative purposes and alternative combinations of ports and/or energy modalities may be possible for the port assembly 412.

As noted above, the modular surgical system 200 can be assembled into different configurations. Further, the different configurations of the modular surgical system 200 can also be utilizable for different surgical procedure types and/or different tasks. For example, FIGS. 4A and 4B illustrate a first illustrative configuration of the modular surgical system 200 including the header module 202 (including the display screen 206) and the energy module 204 connected together. Such a configuration can be suitable for laparoscopic and open surgical procedures, for example. As shown in FIG. 4B, the modular surgical system 200 can be positioned on a cart 230 enabling the modular surgical system 200 to be easily moved (wheeled) around the operating room, for example.

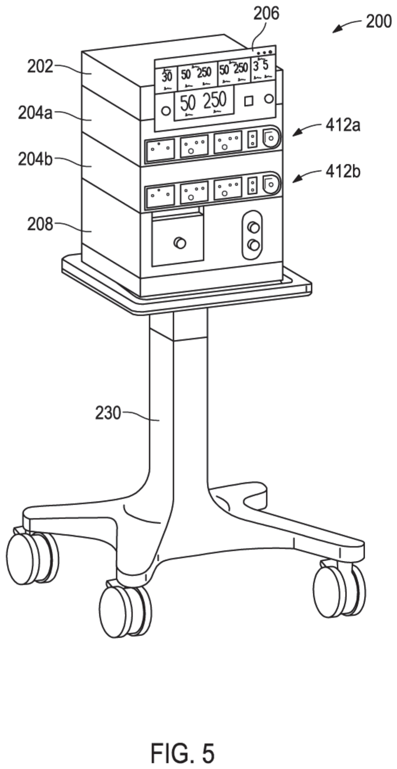

FIG. 5 illustrates a second illustrative configuration of the modular surgical system 200 including the header module 202 (including the display screen 206), a first energy module 204a, a second energy module 204b, and the evacuation module 208 connected together and positioned on the cart 230. In such a configuration, the evacuation module 208 can evacuate smoke, fluid, and/or particulates generated by surgical instruments powered by the energy modules 204a,b.

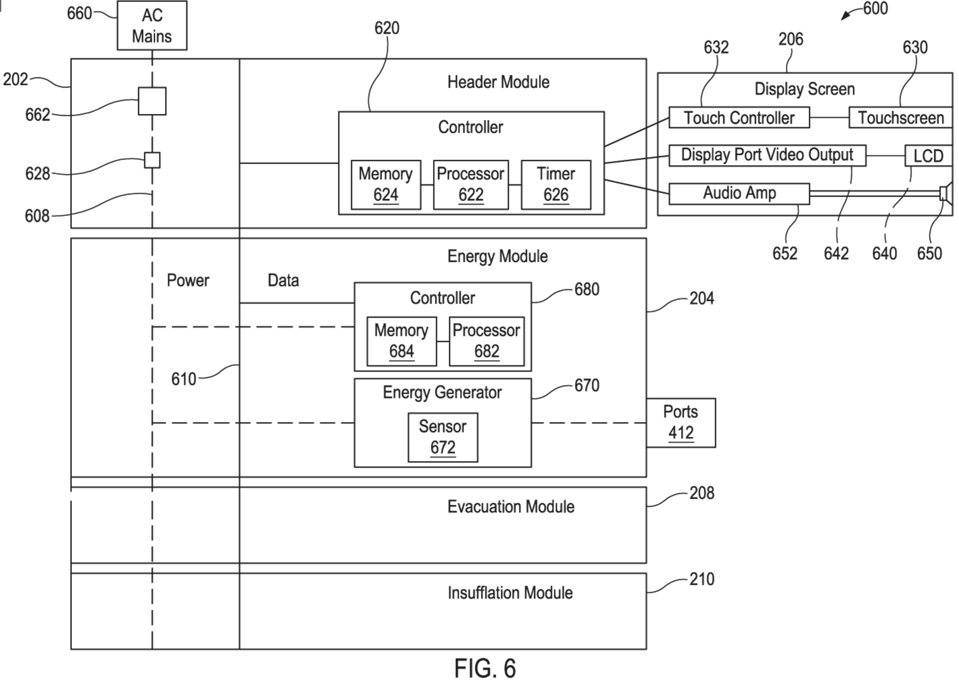

FIG. 6 is a block diagram of an example modular surgical system 600, in accordance with at least one aspect of the present disclosure. As illustrated, the modular surgical system 600 includes the header module 202 (including the display screen 206), the energy module 204 stacked under and coupled to the header module 202, the evacuation module 208 stacked under and coupled to the energy module 204, and the insufflation module 210 stacked under and coupled to the evacuation module 208.

The header module 202 is configured to monitor, control, energize, and provide feedback concerning operation of the modules within the modular surgical system 600, such as the energy module 204, the evacuation module 208, and the insufflation module 210. As illustrated, the header module 202 includes a controller 620 that comprises a processor 622 and a memory 624 storing computer readable instructions executable by the processor 622 to carry out functions and operations of the header module 602. Examples of the memory 624 include, but are not limited to, random access memory (RAM), read-only memory (ROM), computer chips, optical discs (e.g., compact discs (CDs), digital video discs (DVDs), etc.), magnetic disks (e.g., hard disk drives (HDDs), floppy disks, ZIP® disks, etc.), magnetic tape, and solid state storage devices (e.g., memory cards, "flash" media, etc.). As used herein, the term "computer readable medium" refers to any device or system for storing and providing information (e.g., data and instructions) to the processor 622. Examples of computer readable media include, but are not limited to, optical discs, magnetic disks, magnetic tape, solid-state media, and servers for streaming media over networks.

Based on instructions stored in the memory 624, the processor 622 may be configured to control power and data transmissions between the header module 202, the energy module 204, the evacuation module 208, and the insufflation module 210 through a power interface 608 and a data interface 610. For example, the header module 202 can transmit various commands to the energy module 204, the evacuation module 208 (through the energy module 204), and the insufflation module 210 (through the energy module 204 and the evacuation module 208) via the data interface 610. Such commands can be based on user inputs received at the display screen 206 or inputs received by the controller 620 from various sensors communicably coupled to the modular surgical system 600, as discussed elsewhere herein.

As a further example, power may be transmitted to the energy module 204, the evacuation module 208 (through the energy module 204), and the insufflation module 210 (through the energy module 204 and the evacuation module 208) from the header module 202 via the power interface 608. The header module 202 may receive power from an external power source 660 (referred to herein as "AC Mains"), such as a wall outlet, for example. The header module 202 may include an AC/DC converter 662 which receives the AC power from the AC Mains 660 and converts the AC power to DC power. The controller 202 may then distribute the DC power to the energy module 204, the evacuation module 208, and the insufflation module 210 via the power interface 608. The controller 620 may further include a timer 626 for measuring elapsed time. The header module 202 may include a sensor 628, such as a current sensor and/or a power sensor, for example, in operable communication with the controller 620 for measuring current and power along the power interface 608.

As shown in FIG. 6, the energy module 204 may include a controller 680 that comprises a processor 682 and a memory 684 storing computer readable instructions executable by the processor 682 to carry out functions and operations of the energy module 204. The processor 682 and a memory 684 may be similar to processor 622 and memory 624, respectively. The controller 680 may receive power from the AC/DC converter 662 along the power interface 608 and may be in operable communication with controller 620 via the data interface 610.

The energy module 204 may further include an energy generator 670. The energy generator 670 may receive power from the AC/DC converter 662 along the power interface 608 and may be in operable communication with controller 680, such as via a wired or wireless connection. The energy generator 670 may be operable to provide therapeutic energy to one or more surgical instruments, such as the surgical instruments 300, 330, 360, via the port assembly 412, such as via the bipolar port 414 (FIG. 4), the first or second monopolar ports 416a, 416b (FIG. 4), or the combination energy port 420 (FIG. 4), for example. For instance, the energy generator 670 may be energized with DC power provided thereto from the AC/DC converter 662 along the power interface 608. The controller 680 may then receive an input, such as from the controller 620. Based on the input, the controller 680 may control the energy generator 670 to provide therapeutic energy to one or more surgical instruments coupled to the energy module 204 at the port assembly 412. The energy generator 670 may include a sensor 672, such as a current sensor and/or a power sensor, for example, in operable communication with the controller 680 for measuring current and/or power provided by the energy generator 670. The sensor 672 may also comprise an impedance sensor for measuring the impedance of tissue grasped by one of the surgical instruments.

As shown in FIG. 6, the display screen 206 includes a touchscreen 630 coupled to a touch controller 632. The touch controller 632 is coupled to the controller 620 to read inputs, such as user inputs, from the touchscreen 630. The controller 620 drives an LCD display 640 through a display/port video output signal 642. The controller 620 is further coupled to an audio amplifier 652 to drive one or more speakers 650.

*Surgical Insufflation*

Minimally invasive surgical procedures often require the creation of a gas-filled cavity to provide surgeons with adequate visibility and space to manipulate instruments, such as the energy delivery devices 300, 330, 360 of FIG. 3. This gas-filled cavity may be generated using an insufflation module, such as insufflation module 210 (FIG. 2).

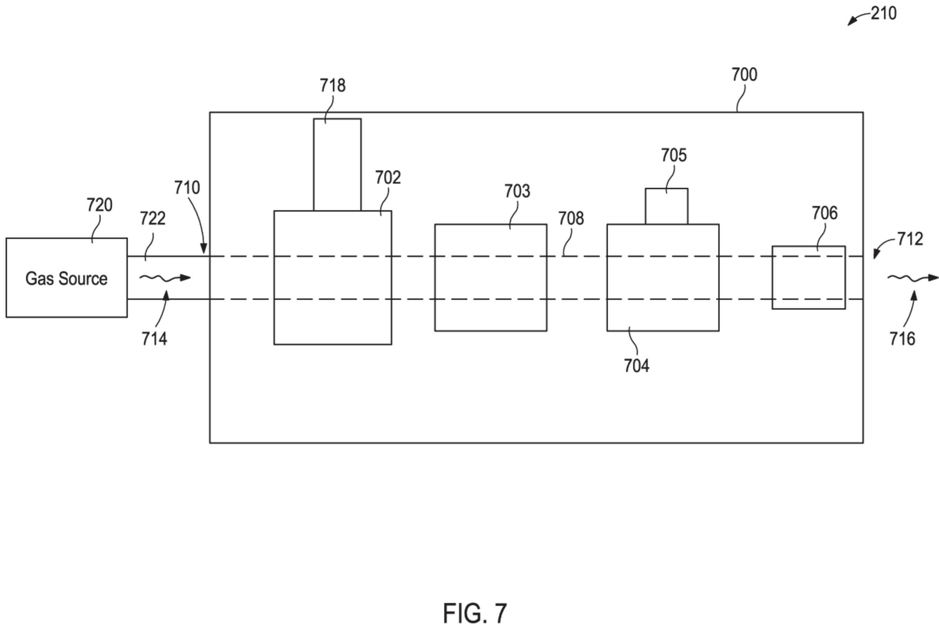

FIG. 7 is a schematic view of the internal components of the insufflation module 210 of FIG. 2, according to at least one aspect of the present disclosure. The insufflation module 210 may include an insufflation housing 700 that contains a fan or pump 702, a humidifier 703, a heat reservoir 704, a heater 705, and an exhaust mechanism 706, all positioned therewithin. In some embodiments, the heater 705 may be operable to generate heat and the heat reservoir 704 is configured to receive and store the heat generated by the heater 705. This stored heat may be used to passively warm gas that moves through the insufflation module 210, as will be described in more detail below. Alternatively, in other embodiments, the heat reservoir 704 may be omitted and the heater 705 may be operable to actively warm gas that moves through the insufflation module 210. The humidifier 703 may be operable to humidify the gas that moves through the insufflation module 210, as will also be described in more detail below. A motor 718 is provided to drive the pump 702. When the insufflation module 210 is stacked with the header module 202, similar to the arrangement shown in FIG. 6, the motor 718, the humidifier 703, and the heater 705 may receive power via the power interface 608.

The insufflation module 210 defines a flow path 708 (shown partially in dashed lines) extending through the insufflation housing 700 and having an inlet port 710 and an outlet port 712. The pump 702, the humidifier 703, the heat reservoir 704, and the exhaust mechanism 706 are sequentially arranged in-line within the flow path 708 through the insufflation housing 700 between the inlet port 710 and the outlet port 712. The outlet port 712 may be fluidically coupled to a trocar, which may be in fluid communication with an internal cavity of a patient (e.g., a patient's peritoneal cavity).

The inlet port 710 may be fluidically coupled to a gas source 720 with a conduit 722 (tubing). The gas source 720 may contain a gas, such as carbon dioxide $(CO_2)$, nitrous oxide $(N_2O)$, helium, oxygen, air, xenon, argon, or nitrogen $(N_2)$, as examples.

The pump 702 is configured to produce a pressure differential in the flow path 708 by a mechanical action. The pressure differential draws gas 714 from the gas source 720 through the conduit 722, into the inlet port 710, and along the flow path 708. After moving through the humidifier 703 and the heat reservoir 704, the gas 714 can be considered "heated/humidified" gas 716 (referred to herein as "insufflation" gas 716), which can continue through the flow path 708 and the exhaust mechanism 706, eventually being expelled (discharged) through the outlet port 712. The exhaust mechanism 706 may control the velocity, the direction, and/or other properties of the insufflation gas 716 exiting the insufflation module 210 at the outlet port 712.

The flow path 708 through the insufflation module 210 can be comprised of a tube or other conduit that substantially contains and/or isolates the fluid moving through the flow path 708 from the fluid (the ambient environment) outside the flow path 708.

*Surgical Smoke Evacuation*

As provided herein, energy devices, such as the energy delivery devices 300, 330, 360 of FIG. 3, deliver mechanical (ultrasonic, for example) and/or electrical (RF, for example) energy to target tissue in order to treat the tissue (e.g. to cut the tissue, cauterize blood vessels and/or coagulate the tissue within and/or near the targeted tissue). Cutting, cauterization, and/or coagulation of tissue can result in fluids and/or particulates being released into the air. Such fluids and/or particulates emitted during a surgical procedure can constitute smoke, for example, which can comprise carbon particles and/or other particles suspended in air. In other words, a fluid can comprise smoke and/or other fluidic matter.

Approximately 90% of endoscopic and open surgical procedures generate some level of smoke. The smoke can be unpleasant to the olfactory senses of the clinician(s), the assistant(s), and/or the patient(s), may obstruct the clinician(s)'s view of the surgical site, and may be unhealthy to inhale in certain instances. For example, smoke generated during an electrosurgical procedure can contain toxic chemicals including acrolein, acetonitrile, acrylonitrile, acetylene, alkyl benzenes, benzene, butadiene, butene, carbon monoxide, creosols, ethane, ethylene, formaldehyde, free radicals, hydrogen cyanide, isobutene, methane, phenol, polycyclic aromatic hydrocarbons, propene, propylene, pyridene, pyrrole, styrene, toluene, and xylene, as well as dead and live cellular material (including blood fragments), and viruses. Certain materials identified in surgical smoke have been classified as containing known carcinogens. It is estimated that one gram of tissue cauterized during an electrosurgical procedure can be equivalent to the toxins and carcinogens of six unfiltered cigarettes. Additionally, exposure to the smoke released during an electrosurgical procedure has been reported to cause eye and lung irritation to health care workers.

In addition to the toxicity and odors associated with the material in surgical smoke, the size of particulate in surgical smoke can be harmful to the respiratory system of the clinician(s), the assistant(s), and/or the patient(s). In certain instances, the particulates can be extremely small, and repeated inhalation of extremely small particulate can lead to acute and chronic respiratory conditions in certain instances.

Many electrosurgical systems employ a surgical evacuation system that draws in and captures the resultant smoke from a surgical procedure, and directs the captured smoke through a filter and an exhaust port away from the clinician(s) and/or from the patient(s). For example, an evacuation system, such as the evacuation module 208 (FIG. 2), can be configured to evacuate smoke that is generated during an electrosurgical procedure. Such an evacuation system can be referred to as a "smoke evacuation system," though such evacuation systems can be configured to evacuate more than just smoke from a surgical site.

Throughout the present disclosure, the "smoke" evacuated by an evacuation system is not limited to just smoke. Rather, the smoke evacuation systems disclosed herein can be used to evacuate a variety of fluids, including liquids, gases, vapors, smoke, steam, or combinations thereof. The fluids can be biologic in origin and/or can be introduced to the surgical site from an external source during a procedure. The fluids can include water, saline, lymph, blood, exudate, and/or pyogenic discharge, for example. Moreover, the fluids can include particulates or other matter (e.g. cellular matter or debris) that is evacuated by the evacuation system. For example, such particulates can be suspended in the fluid.

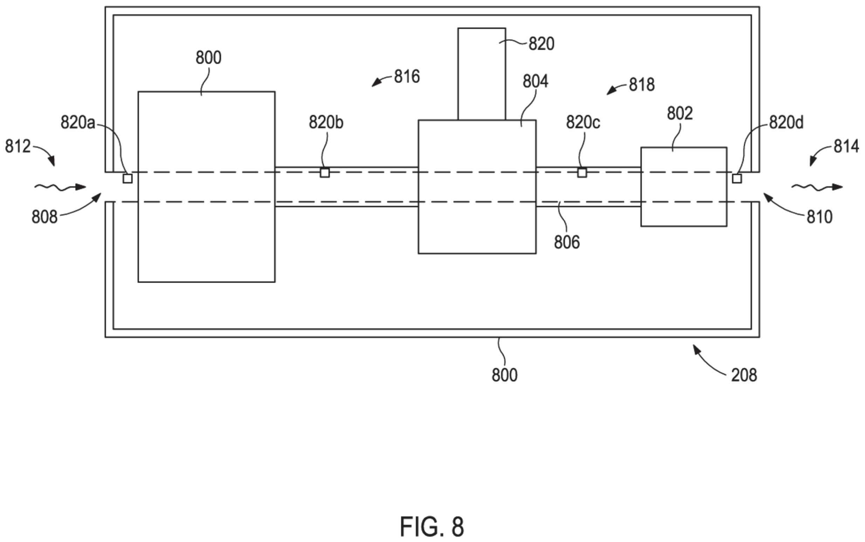

FIG. 8 is a schematic view of the internal components of the evacuation module 208 of FIG. 2, according to at least one aspect of the present disclosure. The evacuation module 208 includes an evacuation housing 801 with a fan or pump 804 and a filter 800 positioned therewithin. Smoke drawn into the evacuation housing 801 travels to the filter 800, and harmful toxins and offensive smells are filtered out of the smoke as it moves through (traverses) the filter 800. Filtered air 814 may then exit the evacuation module 208 as exhaust.

The evacuation module 208 defines a flow path 806 (shown in dashed lines) extending through the evacuation housing 801 and having an inlet port 808 and an outlet port 810. The filter 800, the pump 804, and the exhaust mechanism 802 are sequentially arranged in-line within the flow path 806 through the evacuation housing 801 between the inlet port 808 and the outlet port 810. The inlet port 808 may be fluidically coupled to a suction conduit 702, which can comprise a distal conduit opening positionable at a surgical site.

The pump 804 is configured to produce a pressure differential in the flow path 806 by a mechanical action. The pressure differential is configured to draw smoke 812 from the surgical site into the inlet port 808 and along the flow path 806. After moving through the filter 800, the smoke 812 can be considered "filtered" smoke or air 814 (referred to herein as "filtered air 814"), which can continue through the flow path 806 and is eventually expelled (discharged) through the outlet port 810.

As illustrated, the flow path 806 may include a first zone 816 and a second zone 818. The first zone 816 is located upstream from the pump 804; the second zone 818 is located downstream from the pump 804. The pump 804 is configured to generate the vacuum and otherwise pressurize the fluid in the flow path 806 to drive the fluid from the first zone 816 to the second zone 818 through the pump 804. A motor 820 drives the pump 804. When the evacuation module 208 is stacked with the header module 202, similar to the arrangement shown in FIG. 6, the motor 820 may receive power via the power interface 608. The exhaust mechanism 802 is a mechanism that can control the velocity, the direction, and/or other properties of the filtered air 814 exiting the evacuation module 208 at the outlet port 810.

The flow path 806 through the evacuation module 208 can be comprised of a tube or other conduit that substantially contains and/or isolates the fluid moving through the flow path 806 from the fluid (the ambient environment) outside the flow path 806. For example, the first zone 816 of the flow path 806 can comprise a tube through which the flow path 806 extends between the filter 800 and the pump 804. The second zone 818 of the flow path 806 can also comprise a tube (conduit) through which the flow path 806 extends between the pump 804 and the exhaust mechanism 802. The flow path 806 also extends through the filter 800, the pump 804, and the exhaust mechanism 802 so that the flow path 806 extends continuously from the inlet port 808 to the outlet port 810.

In operation, the smoke 812 can flow into the filter 800 after traversing the inlet port 808 and can be pumped through the flow path 806 by the pump 804 such that the smoke 812 is drawn into the filter 800. The filtered air 814 discharged from the filter 800 can then be pumped through the exhaust mechanism 802 and out the outlet port 810 of the evacuation module 208. The filtered air 814 exiting the evacuation module 208 at the outlet port 810 is the exhaust, and can consist of filtered gases that have passed through the evacuation module 208. Additional information regarding the evacuation module 208 is described in U.S. Patent No. 11,602,393, entitled "SURGICAL EVACUATION SENSING AND GENERATOR CONTROL", issued March 14, 2023, which is hereby incorporated by reference in its entirety herein.

According to embodiments of the present disclosure, the evacuation module 208 may further include a plurality of sensors 820a, 820b, 820c, 820d positioned along the flow path 806 for measuring one or more parameters associated with the smoke 812 and/or the filtered air 814 flowing along the flow path 806. When the evacuation module 208 is in a stacked configuration with a header module 202, such as in the configuration shown in FIG. 6, for example, the sensors 820a-d can be in operable communication with the controller 620, such as along the data interface 610 (FIG. 6), and can receive power from the header module 202 via the power interface 608 (FIG. 6). The controller 620 may receive measurements from the sensors 820a-d and control various operations of the modular surgical system based on these measurements. For example, in some embodiments, the controller 620 can control a speed of the motor 820 based on the received measurements.

One or more of the sensors 820a-d may comprise flow sensors for measuring a flow rate of the smoke 812/filtered air 814 along the flow path 806, such as a flow rate entering the inlet port 808 (sensor 820a), a flow rate through the first zone 816 (sensor 820b), a flow rate through the second zone 818 (sensor 820c), and/or a flow rate exiting the outlet port 810 (sensor 820d), for example. Alternatively, or in addition thereto, one or more of the sensors 820a-d may comprise pressure sensors for measuring a pressure of smoke 812/filtered air 814 along the flow path 806, such as at the inlet port 808 (sensor 820a), the first zone 816 (sensor 820b), the second zone 818 (sensor 820c), and/or at the exit outlet port 810 (sensor 820d), for example. In some embodiments, the sensors 820a-d may be used by the controller 620 to measure a pressure differential along the flow path 806, such as a pressure differential across the filter 800 using the first and second sensors 820a,b. The evacuation module 208 may include a combination of both flow sensors and pressure sensors.

*Using Procedure Checklists to Anticipate Smoke Evacuation Needs*

A procedure checklist may be utilized to guide clinicians and other operating room (OR) staff through a surgical procedure. These checklists may include steps to perform for a given surgical procedure. Smoke needs for a surgical procedure may be anticipated depending on a given step of the surgical procedure.

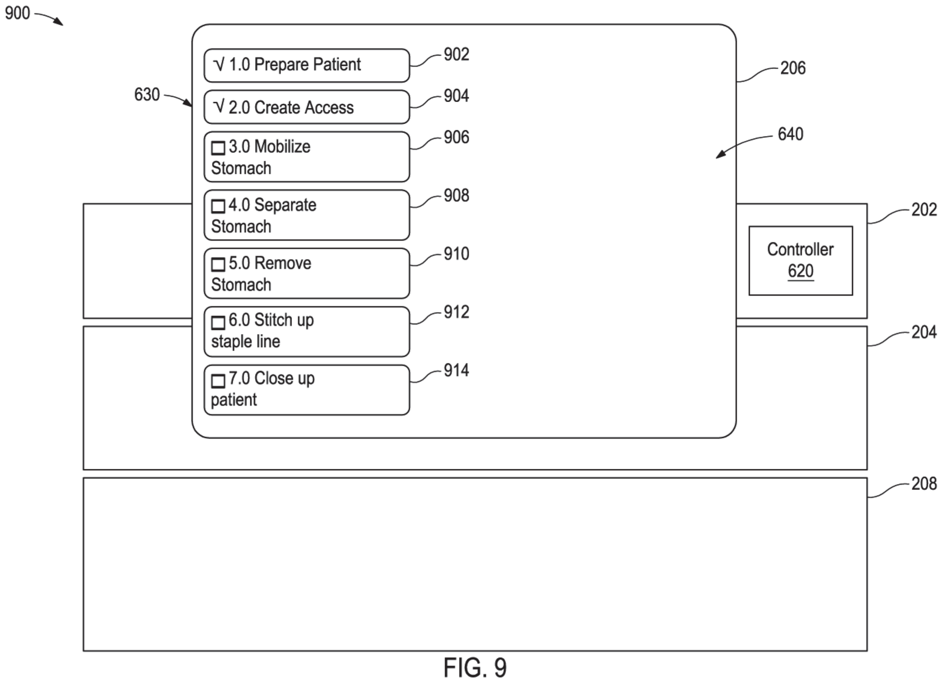

FIG. 9 is a block diagram of an example modular surgical system 900, in accordance with at least one aspect of the present disclosure. As illustrated, the modular surgical system 900 includes a header module 202, a display screen 206 coupled to the header module 202, an energy module 204 stacked under and coupled to the header module 202, and an evacuation module 208 stacked under and coupled to the energy module 204. The modular surgical system 900 may include additional modules, described elsewhere herein, such as an insufflation module 208, for example. The modular surgical system 900 may be similar in some respects to the modular surgical system 600 of FIG. 6, and therefore may be best understood with reference thereto, where like numerals will correspond to like components not described again in detail.

The display screen 206 may include a touchscreen 630 coupled to a controller 620 of the header module 210 to read inputs, such as user inputs, from the touchscreen 630. The controller 620 may drive an LCD display 640 of the display screen 206 based on the user inputs and/or data stored in the memory 624 (FIG. 6) of the controller 620. A user may provide an input to the touchscreen 630 indicative of a type of procedure to be performed. The controller 620 may then retrieve, from the memory 624 (FIG. 6), steps associated with the type of procedure to be performed. The controller 620 may then display, on the LCD display 640, the associated steps of the surgical procedure to be performed.

For instance, with continued reference to FIG. 9, a user may provide an input to the touchscreen 630, informing the controller 620 that a gastrectomy is to be performed. The controller 620 may retrieve the steps associated with a gastrectomy from the memory 624 (FIG. 6) and display the associated steps on the display screen 206 as interactable widgets. The steps may include preparing the patient (widget 902), creating an access point into the patient (widget 904), such as via a trocar, mobilizing the patient's stomach (widget 906), such as with one of surgical devices 300, 330, 360 (FIG. 3) coupled to the energy module 204, separating the stomach from a remaining portion of the stomach (widget 908), such as with one of surgical devices 300, 330, 360 (FIG. 3) coupled to the energy module 204, removing the separated stomach portion from the patient (widget 910), stitching up the cut line on the remaining portion of the stomach (widget 912), and closing up the patient (widget 914).

The clinicians and other OR staff may navigate through the steps of the surgical procedure by providing inputs to the touchscreen 630. An OR staff member may interact with the widgets 902, 904, 906, 908, 910, 912, 914 based on the associated step of the procedure being completed or about to be started. For instance, as shown in FIG. 9, an OR staff member has interacted with (provided an input to) widgets 902, 904, thereby signaling to the controller 620 that the patient has been prepared (via widget 902) and an access point has been created (via widget 904), and that the next step of the procedure to be performed is mobilizing the stomach (widget 906). Accordingly, the controller 620 may be aware of what step of the surgical procedure is next to occur and may, therefore, be able to anticipate upcoming smoke needs, as will be discussed in more detail below.

In addition to the above, or in the alternative, the controller 620 may identify a step of the surgical procedure using a situational awareness module, described in more detail in U.S. Patent No. 11,424,027, entitled "METHOD FOR OPERATING SURGICAL INSTRUMENT SYSTEMS", which issued on August 23, 2022, the contents of which are hereby incorporated by reference in their entirety herein. For instance, the controller 620 may infer that the clinician is in the process of mobilizing the patient's stomach based on the controller 620 receiving data from energy module 204, indicating that an energy device, such as one of surgical instruments 300, 330, 360 (FIG. 3), has been energized. The controller 620 may cross-reference the received data with the retrieved steps of the surgical procedure to determine that an energy device being energized at this point in the procedure (*i.e.,* after the completion of the creating access point step (widget 904)) corresponds to the mobilization step.

The controller 620 may utilize the known steps of the surgical procedure to set parameters of one or more modules in the modular surgical system 900, such as the evacuation module 208. In a gastrectomy, for example, mobilizing the stomach (widget 906) may be expected to generate a first amount of smoke as the surgeon is typically taking fuller "bites" of thick tissue with the surgical instrument. As the surgeon moves to separating the stomach (widget 908), a second amount of smoke less that the first amount of smoke may be expected to be generated as smaller, more isolated bites of tissue are taken with the surgical instrument.

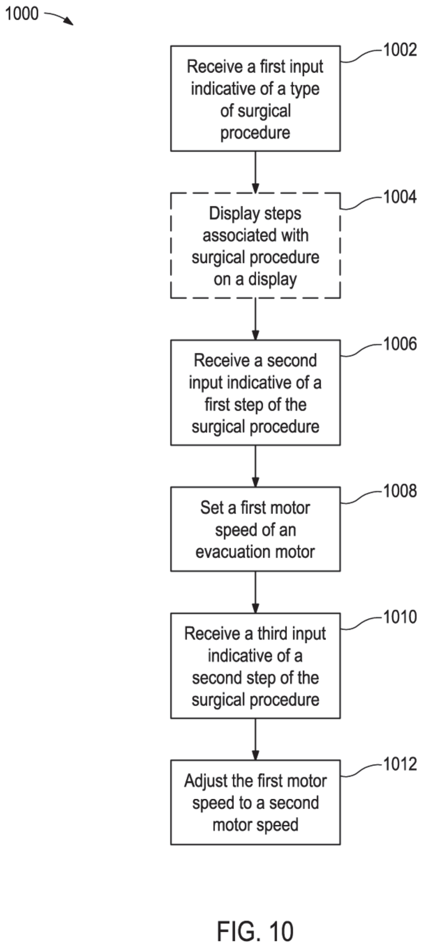

FIG. 10 is a schematic flow chart of an example method 1000 of controlling the modular surgical system 900 of FIG. 9, according to at least one aspect of the present disclosure. The method 1000 may be embodied as an algorithm, stored in the memory 624 (FIG. 6) of the controller 620 (FIG. 9), and may be executable by the processor 622 (FIG. 6) of the controller 620 based on a user providing an input to the controller 620, such as at the touchscreen 630 (FIG. 9).

With reference to FIGS. 9 and 10, the method 1000 may include receiving a first input indicative of a type of surgical procedure, as at step 1002. For instance, a user may provide an input to the controller 620 via the touchscreen 630, informing the controller 620 that a type of surgical procedure, such as a gastrectomy, is to be performed.

The method 1000 may optionally include displaying steps associated with the type of surgical procedure on a display, as at step 1004. For instance, based on receiving the first input, the controller 620 may retrieve, from the memory 624 (FIG. 6), the steps associated with the type of surgical procedure and display, on the display screen 206, the steps as interactable widgets, such as widgets 902-914, informing the clinicians and other OR staff of the steps to be performed for the selected type of surgical procedure.

The method 1000 may further include receiving a second input indicative of a first step of the surgical procedure, as at step 1006. For instance, the controller 620 may receive an input based on a user interacting with one of the displayed widgets 902-914. In the alternative, as discussed above, the controller 620 may automatically receive an input from a situational awareness module. The first step of the surgical procedure may be preparing the patient (widget 902), creating the access point in the patient (widget 904), or mobilizing the stomach (widget 906), as examples.

The method 1000 may further include setting a first motor speed of an evacuation motor, as at step 1008. For instance, the memory 624 (FIG. 6) of the controller 620 may include a look-up table that correlates steps of surgical procedures with preselected motor speeds of the evacuation motor 820 (FIG. 8). The controller 620 may retrieve, from the memory 624, the first motor speed of the evacuation motor 820 based on receiving the second input indicative of the first step of the surgical procedure. The controller 620 may then set the motor speed of the evacuation motor 820 to the first motor speed. The selected motor speed may be sufficient to evacuate an amount of smoke expected to be generated during mobilization of the stomach. The controller 620 may energize the evacuation motor 820 at the first motor speed, such as via the power interface 608 (FIG. 6).

The method 1000 may further include receiving a third input indicative of a second step of the surgical procedure, as at step 1010. For instance, the controller 620 may receive an input based on a user interacting with one of the displayed widgets 902-914 or automatically from a situational awareness module, as discussed above. The second step of the surgical procedure may be separating the stomach for a gastrectomy, for example.

The method 1000 may further include adjusting the first motor speed to a second motor speed, as at step 1012. For instance, the controller 620 may retrieve, from the look-up table in the memory 624, the second motor speed of the evacuation motor 820 based on receiving the third input indicative of the second step of the surgical procedure. The controller 620 may then adjust the motor speed of the evacuation motor 820 from the first motor speed to the second motor speed, which may be different than (less than or greater than) the first motor speed. For example, the second motor speed may be slower than what was used for mobilizing the stomach as less smoke is expected to be generated as the stomach is separated. The controller 620 may energize the evacuation motor 820 at the second motor speed, such as via the power interface 608 (FIG. 6).

The method 1000 may further include receiving a fourth input and adjusting the motor speed to a user selected motor speed based on the fourth input. For instance, after the controller 620 has set the first motor speed (step 1008) or second motor speed (step 1012), a user may desire to increase or decrease the motor speed. A user may provide an input to the controller 620 via the touchscreen 630, informing the controller 620 that a motor speed adjustment to the evacuation motor 820 (FIG. 8) is desired. The controller 620 may adjust the set motor speed (first motor speed, as at step 1008, or second motor speed, as at step 1012) to a user selected motor speed based on the received user input. Therefore, the controller 620 may set motor speeds of the evacuation motor 820 based on a determined step of a surgical procedure and then may adjust the set motor speed based on a user input.

Accordingly, by knowing the type and steps of a surgical procedure being performed, the controller 620 may create initial setpoints at the beginning of the case based upon the most common settings, and secondly, it can adjust settings for each procedure step to account for the change in device usage and consequently smoke generated. Actively adjusting the motor speed of the evacuation motor 820 may also increase the life span of the filter 800 (FIG. 8) by customizing the smoke evacuation and only using the flow rate that is necessary, instead of a one size fits all approach.

### Determining Smoke Evacuation Rates Based on Tissue Classification or Inputs from a Visualization Module

Referring again to FIG. 9, different tissue types typically generate different levels of smoke as they are being operated on (treated) by energy devices, such as one of the surgical instruments 300, 330, 360 (FIG. 3). For example, liver tissue is expected to generate dense, moist smoke as is it treated, so a first (high) flow rate and a first (long) time from the evacuation module 208 may be needed. On the other hand, isolated, thin vessels, such as the thyrocervical trunk, are expected to generate light, aerosolized smoke mist as they are treated, so a second (low) flow rate, less than the first (high) flow rate, and a second (short) time, less than the first (long) time from the evacuation module 208 may be needed. Accordingly, the controller 620 may set parameters for various surgical modules within the modular surgical system 900, such as the evacuation module 208, according to a determined (known) type of tissue being operated on by a surgical instrument.

During an initial period of time at which the surgical instrument, such as one of surgical instruments 300, 330, 360 (FIG. 3), is energized while interacting with tissue, three radiofrequency (RF) electrical parameter measurements may be taken. These electrical parameters may include initial RF impedance, minimum RF impedance, and the amount of time at which the RF impedance slope is approximately 0. The surgical instrument may be coupled to the energy module 204, which may collect and send these measurements to the controller 620 of the header module 202, such as via the data interface 610. Based on this data, the controller 602 may classify (determine) a type of tissue that the surgical instrument is interacting with, such as using a Support Vector Machine (SVM) or another classification algorithm. More information regarding tissue classification using measurements from a surgical instrument can be found in U.S. Patent No. 11,298,148, entitled "LIVE TISSUE CLASSIFICATION USING ELECTRICAL PARAMETERS", issued April 12, 2022, which is hereby incorporated by reference in its entirety herein.

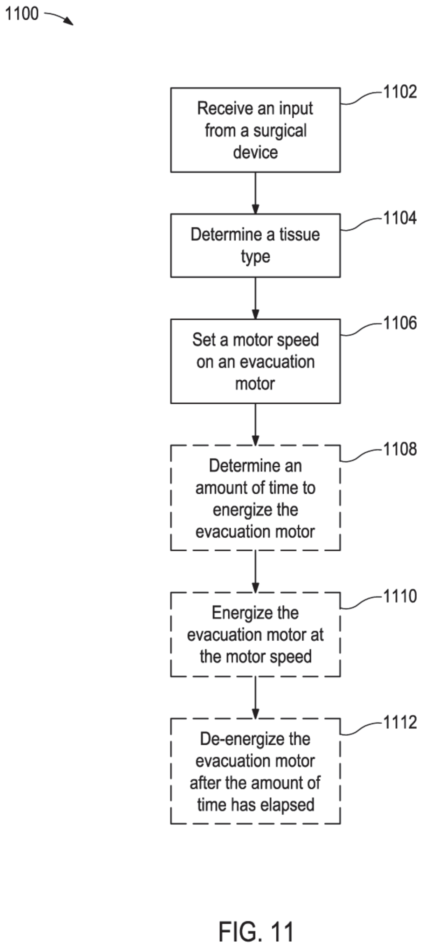

FIG. 11 is a schematic flowchart of an example method 1100 of controlling the modular surgical system 900 of FIG. 9, according to at least one aspect of the present disclosure. The method 1100 may be embodied as an algorithm, stored in the memory 624 (FIG. 6) of the controller 620 (FIG. 9), and may be executable by the processor 622 (FIG. 6) of the controller 620 based on a user providing an input to the controller 620, such as at the touchscreen 630 (FIG. 9).

With reference to FIGS. 9 and 11, the method 1100 may include receiving an input from a surgical device, as at step 1102. In one aspect, the surgical device may be a surgical instrument, such as one of surgical instruments 300, 330, 360 (FIG. 3), and the energy module 204 may receive an input based on the surgical instrument interacting with a tissue. This input may include initial RF impedance, minimum RF impedance, or the amount of time at which the RF impedance slope is approximately 0, or combinations thereof. The controller 620 may receive these inputs from the energy module 204, such as via the data interface 610. In another aspect, the surgical device may comprise a camera and the visualization module 212 (FIG. 2) may receive an input (i.e., imaging data) from camera. The imaging data may include a real-time image of a surgical site within a patient that includes a tissue.

The method 1100 may further include determining a tissue type, as at step 1104. In one aspect, based on the input being received from the surgical instrument/energy module 204, the controller 620 may determine a tissue type using a Support Vector Machine (SVM) or another classification algorithm. In another aspect, based on the input being received from the camera/visualization module 210, the controller 620 may classify the tissue type, such as by comparing the tissue visualized in real-time to images stored in the memory 624 (FIG. 6), or any suitable detection methods described in more detail U.S. Patent No. 11,284,963, entitled "METHOD OF USING IMAGING DEVICES IN SURGERY", issued March 29, 2022, which is hereby incorporated by reference in its entirety herein.

The method 1100 may further include setting a motor speed of an evacuation motor, as at step 1106. For instance, the memory 624 (FIG. 6) of the controller 620 may include a look-up table that correlates types of tissue with preselected motor speeds of the evacuation motor 820 (FIG. 8). The controller 620 may retrieve, from the memory 624, the motor speed of the evacuation motor 820 based on determining the type of tissue. The controller 620 may then set the evacuation motor 820 to the retrieved motor speed and energize the evacuation motor 820 at the set motor speed, such as via the power interface 608 (FIG. 6).

The method 1100 may optionally further include determining a time at which to energize the evacuation motor at the motor speed, as at step 1108. The look-up table in the memory 624 (FIG. 6) may further include an amount of time at which to energize the evacuation motor 820 (FIG. 8) at the selected motor speed. The controller 620 may retrieve, from the memory 624, the motor speed of the evacuation motor 820 and the amount of time at which to energize the evacuation motor 820 (FIG. 8) based on determining the type of tissue.

The method 1100 may optionally further include energizing the evacuation motor at the motor speed, as at step 1110 and de-energizing the evacuation motor based on the amount of time elapsing, as at step 1112. For instance, based on the controller 620 determining an amount of time to energize the evacuation motor 820 (FIG. 8) at step 1108, the controller 620 may energize the evacuation motor 820 at the set motor speed, such as via the power interface 608 (FIG. 6). The controller 620 may further measure an elapsed amount of time, such as with timer 626 (FIG. 6) and de-energize the evacuation motor 820 (FIG. 8) based on the elapsed amount of time reaching the determined amount of time.

In some applications, the method 1100 may further include receiving a second input and adjusting the motor speed to a user selected motor speed based on the second input. For instance, after the controller 620 has set the motor speed (step 1106), a user may desire to increase or decrease the motor speed. A user may provide an input to the controller 620 via the touchscreen 630, informing the controller 620 that a motor speed adjustment of the evacuation motor 820 (FIG. 8) is desired. The controller 620 may then adjust the set motor speed to a user-selected motor speed based on the received user input. Therefore, the controller 620 may set motor speeds of the evacuation motor 820 based on a determined tissue type and then adjust the set motor speed based on a user input.

Accordingly, the controller 620 may be operable to set motor speeds of an evacuation motor to account for smoke that is expected to be generated for a given tissue type. Actively adjusting the motor speed of the evacuation motor 820 may also increase the life span of the filter 800 (FIG. 8) by customizing the smoke evacuation and only using the flow rate that is necessary, instead of a one size fits all approach.

*Using System Feedback to Determine Filter Life*

Over time, filters in smoke evacuators, such as the filter 800 (FIG. 8) in the evacuator module 208 (FIG. 8), can become saturated with airborne particles and their effectiveness may correspondingly diminish, necessitating timely replacement to maintain optimal performance of the evacuator module 208. Current methods for determining when to replace the filter 800 may not adequately quantify the amount of particulate that has passed through the filter 800, which can result in the filter 800 being replaced too early or too late. Accordingly, improved systems and methods for determining when to replace a filter in an evacuation module are desired.

Referring to FIGS. 8 and 9, the controller 620 may receive a plurality of inputs and calculate (determine) a theoretical amount of smoke captured by the filter 800 during a surgical procedure based on the plurality of inputs. The plurality of inputs may include a type of surgical instrument coupled to the energy module 204, such as one of surgical instruments 300, 330, 360 (FIG. 3), a type of surgical procedure being performed or to be performed, such as an open procedure or a laparoscopic procedure, and/or an amount of time that the surgical instrument is energized for. The controller 620 may use the theoretical amount of smoke captured by the filter 800 to calculate (determine) an amount of life remaining of the filter 800. The controller 620 may compare the amount of life remaining to a threshold, which may be stored in the memory 624 (FIG. 6), to determine if the filter 800 may be used in a subsequent surgical procedure, or if it should be replaced.

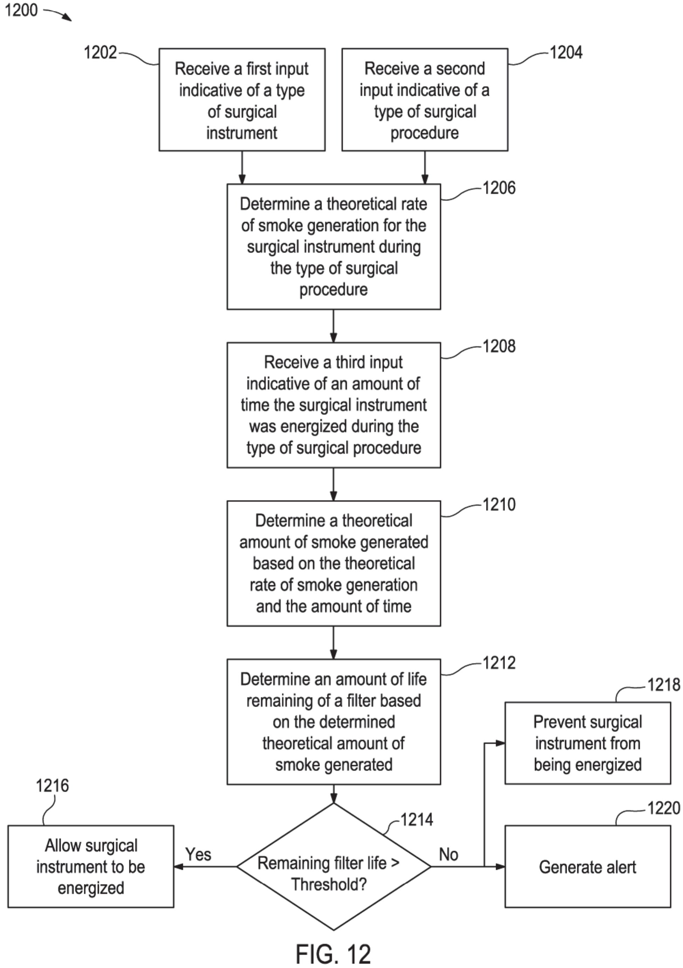

FIG. 12 is a schematic flowchart of an example method 1200 of controlling the modular surgical system 900 of FIG. 9, according to at least one aspect of the present disclosure. The method 1200 may be embodied as an algorithm, stored in the memory 624 (FIG. 6) of the controller 620 (FIG. 9), and may be executable by the processor 622 (FIG. 6) of the controller 620 based on a user providing an input to the controller 620, such as at the touchscreen 630 (FIG. 9).

With reference to FIGS. 9 and 12, the method 1200 may include receiving a first input indicative of a type of surgical instrument, as at step 1202. For instance, the controller 620 may receive the first input based on a surgical instrument, such as one of the surgical instruments 300, 330, 360 (FIG. 3), being coupled to the energy module 204 or based on a user providing an input to the controller 620 via the touchscreen 630 of the display 206. The method 1200 may further include receiving a second input indicative of a type of surgical procedure, as at step 1204. For instance, the controller 620 may receive the second input based on a user providing an input to the controller 620 via the touchscreen 630 or based on an input from a situational awareness module, as described in more detail in U.S. Patent No. 11,424,027, entitled "METHOD FOR OPERATING SURGICAL INSTRUMENT SYSTEMS", which issued on August 23, 2022, the contents of which are hereby incorporated by reference in their entirety herein. The type of surgical procedure may be a general type of surgical procedure, such as an open or laparoscopic surgical procedure, or a specific type of surgical procedure, such as a gastrectomy, for example.

The method 1200 may further include determining a theoretical rate of smoke generation for the surgical instrument during the type of surgical procedure, as at step 1206. The memory 624 (FIG. 6) may include a look-up table that correlates types of surgical instruments and types of surgical procedures to theoretical rates of smoke production. For instance, the look-up table may include a first surgical instrument, such as a monopolar pen, as being expected to generate a first theoretical rate of smoke for a first type of surgical procedure (e.g., a laparoscopic surgical procedure), and a second theoretical rate of smoke different than the first theoretic rate of smoke for a second type of surgical procedure (e.g., an open surgical procedure). An example monopolar pen is shown and described in U.S. Patent Application Publication No. 2010/0036373, titled "ELECTROSURGICAL SYSTEM HAVING A SENSOR FOR MONITORING SMOKE AND AEROSOLS", which published February 11, 2010, the contents of which are hereby incorporated by reference in their entirety herein.

The look-up table may further include a second surgical instrument, such as the RF surgical instrument 330 (FIG. 3), as being expected to generate a third theoretical rate of smoke for the first type of surgical procedure and a fourth theoretical rate of smoke different than the third theoretic rate of smoke for the second type of surgical procedure. The look-up table may further include a third surgical instrument, such as the multifunctional surgical instrument 360 (FIG. 3), as being expected to generate a fifth theoretical rate of smoke for the first type of surgical procedure and a sixth theoretical rate of smoke different than the fifth theoretic rate of smoke for the second type of surgical procedure.

The method 1200 may further include receiving a third input indicative of an amount of time the surgical instrument was energized during the type of surgical procedure, as at 1208. For instance, the controller 620 may receive an input from the energy module 204 based on the surgical instrument being energized to treat tissue. Based on receiving the input, the controller 620 may measure an elapsed amount of time using the timer 626 (FIG. 6). The controller 620 may then, at a later time, receive another input indicating that the surgical instrument is no longer being energized to treat the tissue.

Based on receiving the input indicative of the surgical instrument being no longer energized, the controller 620 may stop the timer 626. The controller 620 may repeat this process (starting and stopping the timer 626) each time the surgical instrument is activated/deactivated to measure the total amount of time that the surgical instrument is energized during the surgical procedure.

The method 1200 may further include determining a theoretical amount of smoke generated based on the theoretical rate of smoke generation and the amount of time, as at step 1210. For instance, the controller 620 may calculate (determine) the theoretical rate of smoke generation by multiplying the theoretical rate of smoke generation, as determined at step 1206, to the amount of time the surgical instrument was energized, as determined at step 1208.

The method 1200 may further include determining an amount of life remaining of a filter based on the determined theoretical amount of smoke generated, as at 1212. More specifically, the controller 620 may store, in the memory 624 (FIG. 6), a filter life value of the filter 800 (FIG. 8) of the evacuation module 208. The filter life value may be provided to the controller 620 by a user at the touchscreen 630 or may be set automatically by the controller 620 based on the filter 800 being placed into the evacuator module 208. Based on the surgical instrument being energized during the surgical procedure, the controller 620 may energize the motor 820 (FIG. 8) of the evacuator module 208 to draw the smoke generated by the surgical instrument through the filter 800 (FIG. 8), as described elsewhere herein. The controller 620 may utilize the determined theoretical amount of smoke generated to track the life of the filter 800 over time.

The controller 620 may retrieve the filter life value from the memory 624 (FIG. 6) and decrement the determined theoretical amount of smoke generated from the filter life value, thereby generating a remaining filter life value, which can be written to (recorded in) the memory 624. The controller 620 may retrieve the remaining filter life value as a starting point when decrementing theoretical amounts of smoke generated for subsequent surgical procedures.

The method 1200 may further include comparing the remaining filter life to a threshold value, as at step 1214. For instance, the controller 620 may retrieve, from the memory 624, a threshold value to compare to the remaining filter life value, as determined at step 1212. The controller 620 may further perform an action based on the comparison. For instance, the method 1200 may further include allowing the surgical instrument to be energized for a subsequent surgical procedure based on the remaining filter life value being at or greater than the threshold value, as at step 1216. Alternatively, the method 1200 may include preventing the surgical instrument from being energized for a subsequent surgical procedure, as at step 1218, and/or generating an alert, as at step 1220, based on the remaining filter life value being less than the threshold value. In such embodiments, the controller 620 may prevent the AC Mains 660 (FIG. 6) from providing power to the surgical instrument based on the remaining filter life value being less than the threshold value. The controller 620 may further generate an alert to inform the user that the filter 800 (FIG. 8) should be replaced. The alert may be a visual alert via the LCD 640 (FIG. 6) or an audio alert via the speaker 650 (FIG. 6).

Accordingly, the foregoing method takes system feedback, such as instrument parameters (e.g., surgical instrument type), operating environment (e.g., open or laparoscopic procedure), and activation times of the surgical instrument, to calculate a theoretical amount of smoke, which may then be correlated to a percentage of the filter life used. This remaining filter life value may be written to the memory 624 (FIG. 6) and subsequently used (referred to) in future surgical procedures to track filter life over time. This approach may extend the life of the filter 800 (FIG. 8) as actual inputs and parameters from surgical procedures are being used, rather than just merely using time as an indicator of filter life.

*Final Evacuation Mode*

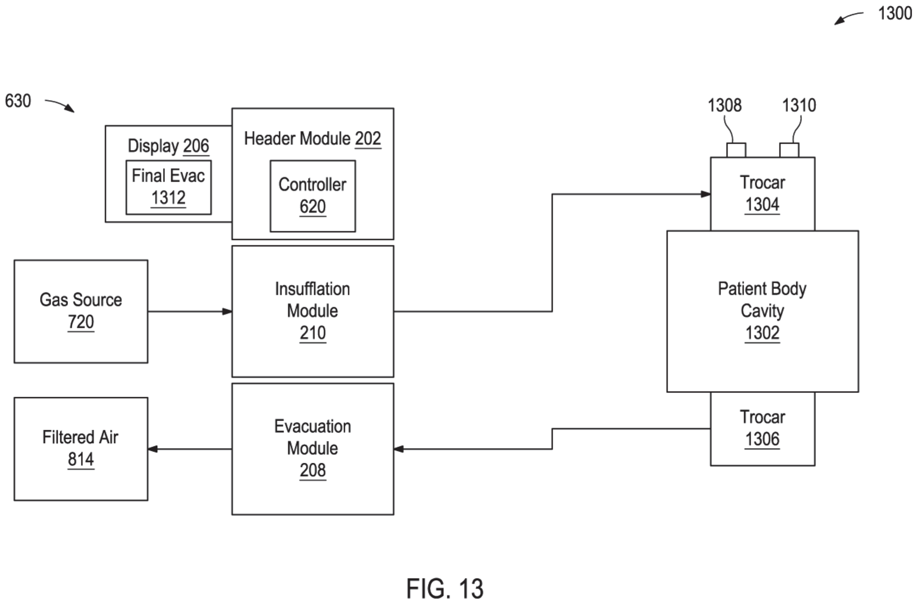

FIG. 13 is a block diagram of another example modular surgical system 1300, in accordance with at least one aspect of the present disclosure. The modular surgical system 1300 may be similar in some respects to the modular surgical system 600 of FIG. 6, and therefore may be best understood with reference thereto. As illustrated, for example, the modular surgical system 1300 includes the header module 202 that includes the controller 620, the display screen 206 coupled to the header module 202, the insufflation module 210 stacked under and coupled to the header module 202, and the evacuation module 208 stacked under and coupled to the insufflation module 210. The modular surgical system 1300 may include additional modules, described elsewhere herein, such as the energy module 204, for example.

The modular surgical system 1300 may further include an insufflation trocar 1304 inserted into a patient's body cavity 1302 and in fluid communication with the insufflation module 210 to receive insufflation gas 716 (FIG. 7) therefrom. The modular surgical system 1300 may further include an evacuation trocar 1306 inserted into the patient's body cavity 1302 and in fluid communication with the evacuation module 208 to draw (evacuate) smoke 812 (FIG. 8) from the patient's body cavity 1302.

The modular surgical system 1300 may further include a pressure sensor 1308 for sensing the pressure within the patient's body cavity 1302 and a flow sensor 1310 for measuring the flow rate of insufflation gas 716 (FIG. 7) from the insufflation module 210 to the patient's body cavity 1302. As illustrated, the pressure and flow sensors 1308, 1310 may be coupled to the insufflation trocar 1304, but could alternatively be located at any suitable location within the modular surgical system 1300, such as at the insufflation module 210 or along the flow line between the insufflation module 210 and the insufflation trocar 1304, as examples. The controller 620 may receive the pressure and flow measurements from the pressure and flow sensors 1308, 1310, respectively, such as via a wireless or wired connection, such as along the data interface 610 (FIG. 6).

In some instances, it may be desirable to evacuate gases in the patient's body cavity 1302 at the conclusion of a surgical procedure, such as prior to removal of the insufflation and evacuation trocars 1304, 1306. Accordingly, the controller 620 may display, on the touchscreen 630, a final evacuation widget 1312 that a clinician may interact with (press) when a final evacuation of the patient's body cavity 1302 is desired.

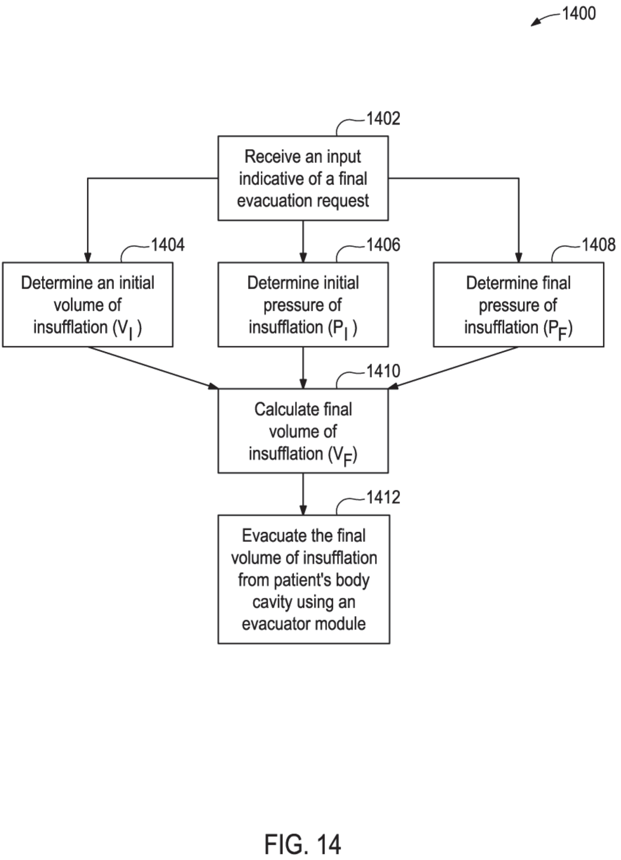

FIG. 14 is a schematic flow chart of an example method 1400 of controlling the modular surgical system 1300 of FIG. 13, according to at least one aspect of the present disclosure. The method 1400 may be embodied as an algorithm, stored in the memory 624 (FIG. 6) of the controller 620 (FIG. 13), and may be executable by the processor 622 (FIG. 6) of the controller 620 based on a user providing an input to the controller 620, such as at the touchscreen 630 (FIG. 13).

With reference to FIGS. 13 and 14, the method 1400 may include receiving an input indicative of a final evacuation request, as at step 1402. For instance, a user may interact with (e.g., press) the final evacuation widget 1312 at the conclusion of a surgical procedure when a final evacuation of gas from the patient's body cavity 1302 is desired. The controller 620 may receive the input based on the user interacting with the final evacuation widget 1312.

The method 1400 may further include determining an initial volume of insufflation ($V_I$), as at step 1404. For instance, the controller 620 may measure an initial volume of insufflation gas 716 (FIG. 7) provided to the patient's body cavity 1302, such as at the beginning of the surgical procedure. The controller 620 may monitor the rate of insufflation gas provided to the patient's body cavity 1302 via the flow sensor 1310 (*e.g.,* in $cm^3$/sec), while also measuring an elapsed amount of time that the insufflation gas 716 is provided to the patient's body cavity 1302 (*e.g.,* in seconds). The controller 620 may calculate the initial volume of insufflation $V_I$ by multiplying the determined rate of insufflation gas and the elapsed amount of time. The controller 620 may store the initial volume of insufflation $V_I$ in the memory 624 (FIG. 6), and subsequently retrieve the initial volume of insufflation $V_I$, as needed.

The method 1400 may further include determining an initial pressure of insufflation ($P_I$), as at step 1406. For instance, the controller 620 may measure the initial pressure of insufflation $P_I$, such as at the beginning of the surgical procedure, via the pressure sensor 1308. The controller 620 may store the initial pressure of insufflation $P_I$ in the memory 624 (FIG. 6), and subsequently retrieve the initial pressure of insufflation $P_I$, as needed.

The method 1400 may further include determining a final pressure of insufflation ($P_F$), as at step 1408. For instance, the controller 620 may measure the final pressure of insufflation $P_F$, such as at the conclusion of the surgical procedure, or based on the user interacting with the final evacuation widget 1312, via the pressure sensor 1308. The controller 620 may store the final pressure of insufflation $P_F$ in the memory 624 (FIG. 6), and subsequently retrieve the final pressure of insufflation $P_F$, as needed.

The method 1400 may further include calculating a final volume of insufflation ($V_F$), as at step 1410. For instance, the controller 620 may calculate the final volume of insufflation $V_F$ using the following expression, which may be stored in the memory 624 (FIG. 6):

$$V_F = P_I * V_I/P_F$$

The method 1400 may further include evacuating the final volume of insufflation $V_F$ from the patient's body cavity using an evacuator module, as at step 1412. In one aspect, the controller 620 may estimate an amount of time needed to energize the motor 820 (FIG. 8) of the evacuation module 208 based on the calculated final volume of insufflation $V_F$ and the known (set) motor current (speed) of the motor 820. The controller 620 may then energize the motor 820 to evacuate the final volume of insufflation $V_F$ from the patient's body cavity 1302 for the estimated amount of time. In another aspect, the controller 620 may energize the motor 820 and measure the flow rate of smoke 812 (FIG. 8) and gas from the patient's body cavity 1302 via the flow sensor 820a (FIG. 8) and measure an elapsed amount of time using the timer 626 (FIG. 6). The controller 620 may de-energize the motor 820 based on the product of the measured rate and the measured time approaching, reaching, or exceeding the final volume of insufflation $V_F$.

Accordingly, the foregoing system and method may reduce the amount of surgical smoke that the OR staff is exposed to as the final volume of smoke within the patient is evacuated prior to trocar removal from the patient.

*Intelligent Insufflation and Smoke Evacuation*

Operating room (OR) staff, such as nurses, typically have to manually adjust multiple settings of insufflators and evacuators, such as run time, pressures, or flow rates, during a surgical procedure as devices are changed and/or different procedural steps are completed. Systems and methods for adjusting settings pre-emptively, or automatically, may provide a more efficient surgical procedure with less technical support needed.

Referring again to FIGS. 6-8, the controller 620 may collect data from a plurality of sources and set motor speeds of the evacuation motor 820 and/or the insufflation motor 718 based on the collected data. The data collected by the controller 620 may include a type of trocar inserted into a patient, a type of trocar accessory, a type of instrument coupled to the energy module 204, such as an ultrasonic, RF, or multifunctional instrument 300, 330, 360 (FIG. 3), a product code of a surgical instrument coupled to the energy module 204, instrument settings of the surgical instrument, such as power, displacement, voltage, or impedance, or combinations thereof, activation times of the surgical instrument, measured by the energy module 204 or the timer 626, a procedural step of a surgical procedure, smoke consistency and/or thickness of the smoke 812 at a surgical site, as measured by the evacuation module 208 or the visualization module 212, for example, the clarity of the patient's body cavity, as detected by a camera of the visualization module 212, for example, or a type of tissue at the surgical site, as visualized by a camera of the visualization module 212 or detected by the header module 202, as discussed elsewhere herein, for example, or combinations thereof.

Based on the collected data, the controller 620 may be operable to automatically adjust the evacuation motor 820 and/or the insufflation motor 718. As an example, the controller 620 may determine that a clinician is using a high displacement setting of the ultrasonic surgical instrument 300 for cutting liver tissue. Accordingly, the controller 620 may increase the speed of the evacuation motor 820 as additional smoke is expected to be generated. As another example, the controller 620 may detect a decrease in abdomen clarity below a clarity threshold, via the camera of the visualization module 210. Accordingly, the controller 620 may automatically energize the evacuation motor 820 and de-energize the same after the clarity has reached or exceeded the clarity threshold. Therefore, each of the foregoing factors may be used by the controller 620 to select appropriate run times, pressures, and flow rates of the evacuation motor 820 and/or the insufflation motor 718 to maximize the performance of the modular surgical system 600.

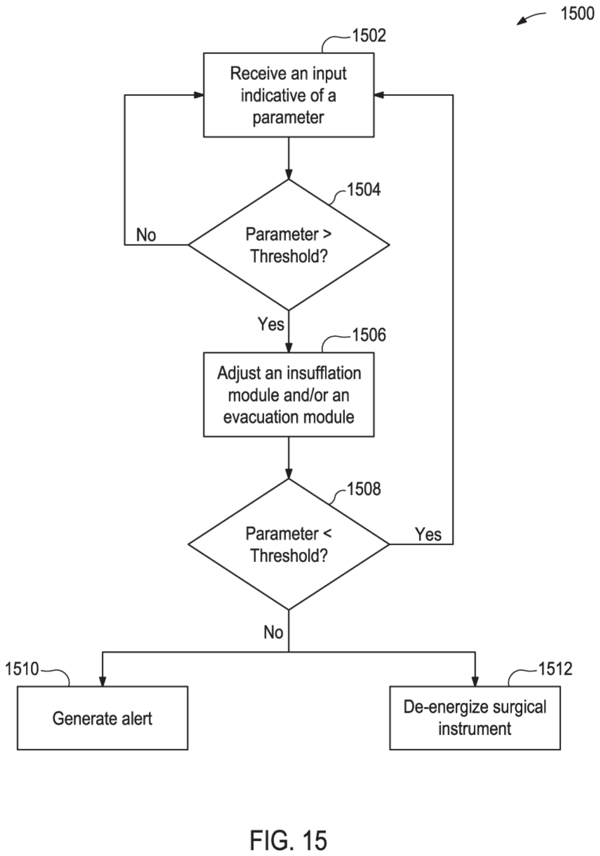

FIG. 15 is a schematic flow chart of an example method 1500 of controlling the modular surgical system 600 of FIG. 6, according to at least one aspect of the present disclosure. The method 1500 may be embodied as an algorithm, stored in the memory 624 (FIG. 6) of the controller 620 (FIG. 6), and may be executable by the processor 622 (FIG. 6) of the controller 620 based on a user providing an input to the controller 620, such as at the touchscreen 630 (FIG. 6).

With reference to FIGS. 6 and 15, the method 1500 may include receiving an input indicative of a parameter, as at step 1502. For instance, the controller 620 may receive a parameter from a data source, such as any of the modules, sensors, instruments, or devices described elsewhere herein. The parameter may be smoke consistency, smoke thickness, amount of smoke, clarity, or any other suitable parameter described elsewhere herein.

The method 1500 may further include comparing the parameter to a threshold, as at step 1504. For instance, the controller 620 may retrieve, from the memory 624, a corresponding threshold and compare a value of the received parameter to the threshold. If the value of the parameter is below the threshold, the controller 620 may loop back to step 1502 and repeat the aforementioned steps of the method 1500 until a value of a parameter exceeds a corresponding threshold.

If the value of the parameter is at or above the threshold, the controller 620 may proceed to adjust an insufflation module and/or an evacuation module, as at step 1506. For instance, when the value of the parameter reaches or exceeds the threshold, the controller 620 may be operable to energize the insufflation module 210 (if de-energized), energize the evacuation module 208 (if de-energized), adjust a parameter of the insufflation module 210 (if already energized), such as the speed of the insufflation motor 718 (FIG. 7), or adjust a parameter of the evacuation module 208 (if already energized), such as the speed of the evacuation motor 820 (FIG. 8), or combinations thereof.

The method 1500 may further include comparing the parameter to the threshold, as at step 1508. For instance, after a threshold amount of time has elapsed from making an adjustment at step 1506, the controller 620 may again compare the value of the parameter to the corresponding threshold. If the value of the parameter is now below the threshold, the controller 620 may loop back to step 1502 and repeat the aforementioned steps of the method 1500.

If the value of the parameter is still at or above the threshold, however, the controller 620 may be operable to generate an alert, as at step 1510, and/or de-energize a surgical instrument, as at step 1512. For instance, based on the value of the parameter still exceeding the threshold despite the adjustment at step 1508, the controller 620 may proceed to de-energize a surgical instrument, such as one of surgical instruments 300, 330, 360, to prevent additional smoke from being generated at the surgical site until a corrective action is taken. The corrective action may include allowing the evacuation module 208 to draw smoke 812 (FIG. 8) from the patient's body cavity without the surgical instrument being activated. The controller 620 may further generate an alert, such as a visual alert via the LCD 640, an audible alert via the speaker 650, and/or a haptic alert via a motor positioned in one of the surgical instruments.

### Use AI to learn Optimal Smoke Evacuator Settings and Performance

A lot is unknown in smoke evacuation in terms of acceptable flow rates, degree of visibility based on settings used, and required time to evacuate smoke. One way to enable smarter smoke removal is by way of artificial intelligence (AI).

Referring again to FIG. 6, the controller 620 of the header module 202 may include an AI platform that collects data from a plurality of data sources, such as any of the modules, sensors, instruments, or devices described elsewhere herein. The AI platform may utilize the collected data to develop optimal settings for the evacuator module 208, such as preselected speeds of the evacuation motor 820 (FIG. 8). The type of data collected by the AI platform may include the type and energy modality of the surgical instrument being utilized, such as one of surgical instruments 300, 330, 360 (FIG. 3), the type of surgical procedure (*e.g.*, open, laparoscopic, gastrectomy, *etc.*), the type of tissue being operated on, the amount of power supplied, or the degree of visibility, or combinations thereof.

Using this information, the AI platform of the controller 620 may become smarter over time by tailoring settings towards procedure and device types to become more efficient. This would allow the surgical staff during pre-op to select a profile on the touchscreen 630 that is optimal for the best visibility and removal of harmful plume for a specific procedure type. The profile may include optimal flow rates, flow times, and activation periods. These profiles may make the modular surgical system 600 more efficient and take the guess work away from the OR staff. This may also help improve set-up times and allow the OR staff to not have to monitor the evacuator module 208 during use.

Embodiments disclosed herein include:

A. A surgical system comprising an evacuation module and a controller. The evacuation module includes a motor and a pump drivable by the motor to draw smoke from a patient. The controller is operable to receive a first input indicative of a first step of a surgical procedure, set a first motor speed of the motor based on the first input, receive a second input indicative of a second step of the surgical procedure, and adjust the first motor speed to a second motor speed different than the first motor speed based on the second input.

B. A surgical system comprising an evacuation module and a controller. The evacuation module includes a motor and a pump drivable by the motor to draw smoke from a patient. The controller is operable to receive an input from a surgical instrument, determine a type of tissue based on the input, and set a motor speed of the motor based on the type of tissue determined.

C. A surgical system comprising an evacuation module and a controller. The evacuation module includes a motor and a pump drivable by the motor to draw smoke from a patient. The controller is operable to receive a first input indicative of a type of surgical instrument, receive a second input indicative of a type of surgical procedure, receive a third input indicative of an amount of time that the surgical instrument was energized during the type of surgical procedure, and determine a theoretical amount of smoke produced during the amount of time.

Each of embodiments A-C may have one or more of the following additional elements in any combination: Element 1: further comprising a display, wherein the controller is operable to receive the first and second inputs via the display. Element 2: wherein the controller is further operable to receive a third input indicative of a type of the surgical procedure and display steps associated with the surgical procedure on the display based on the third input. Element 3: wherein the controller is operable to display the steps associated with the surgical procedure as interactable widgets on the display. Element 4: wherein the controller is operable to receive the first input via a first interactable widget on the display and receive the second input via a second interactable widget on the display. Element 5: wherein the controller is further operable to receive a user input and adjust the first motor speed to a third motor speed different than the first and second motor speeds based on the user input. Element 6: wherein the input comprises an impedance measurement. Element 7: wherein the controller is operable to set a first motor speed of the motor based on a first type of tissue determined and set a second motor speed of the motor different than the first motor speed based on a second type of tissue determined. Element 8: wherein the controller is further operable to determine an amount of time to energize the motor based on the type of tissue determined Element 9: wherein the motor speed is a first motor speed and the controller is further operable to receive a user input and adjust the first motor speed to a second motor speed different than the first motor speed based on the user input. Element 10: wherein the controller is further operable to determine a theoretic rate of smoke generation based on the first and second inputs, the controller being further operable to determine the theoretical amount of smoke produced based on the theoretical rate of smoke generation and the amount of time. Element 11: wherein the evacuation module further comprises a filter, and wherein the controller is further operable to determine an amount of life remaining of the filter based on the theoretical amount of smoke produced. Element 12: wherein the controller is further operable to compare the amount of life remaining of the filter to a threshold and perform an action based on the comparison. Element 13: wherein the action comprises allowing the motor to be energized for a subsequent surgical procedure based on the amount of life remaining being at or above the threshold. Element 14: wherein the action comprises preventing the motor from being energized for a subsequent surgical procedure based on the amount of life remaining being less than the threshold. Element 15: further comprising a display, wherein the action comprises generating an alert on the display. Element 16: wherein the type of surgical procedure is selected from the group consisting of an open surgical procedure and a laparoscopic surgical procedure.

By way of non-limiting example, exemplary combinations applicable to A, B, and C include: Element 1 with Element 2; Element 1 with Elements 2 and 3; Element 1 with Elements 2-4; Element 1 with Elements 2-5; Element 1 with Element 5; Element 6 with Element 7; Element 6 with Element 8; Element 6 with Element 9; Element 6 with two of more of Elements 7-9; Element 7 with Element 8; Element 7 with Element 9; Element 7 with Elements 8 and 9; Element 8 with Element 9;Element 10 with Element 11; Element 10 with Elements 11 and 12; Element 10 with Elements 11-13; Element 10 with Elements 11, 12, and 14; Element 10 with Elements 11, 12, and 15; Element 10 with Elements 11-15; Element 10 with Element 16; Element 10 with two or more of Elements 11-16; Element 11 with Element 12; Element 11 with Elements 12 and 13; Element 11 with Elements 12 and 14; Element 11 with Elements 12 and 15; Element 11 with Element 16; Element 11 with two or more of Elements 12-16.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The use of directional terms such as above, below, upper, lower, upward, downward, left, right, and the like are used in relation to the illustrative embodiments as they are depicted in the figures, the upward direction being toward the top of the corresponding figure and the downward direction being toward the bottom of the corresponding figure.

## Claims

1. A surgical system, comprising:

   an evacuation module that includes a motor and a pump drivable by the motor to draw smoke from a patient; and
   a controller operable to:

   receive a first input indicative of a first step of a surgical procedure;
   set a first motor speed of the motor based on the first input;
   receive a second input indicative of a second step of the surgical procedure; and
   adjust the first motor speed to a second motor speed different than the first motor speed based on the second input.

2. The surgical system of Claim 1, further comprising a display, wherein the controller is operable to receive the first and second inputs via the display; optionally wherein the controller is further operable to:

   receive a third input indicative of a type of the surgical procedure; and
   display steps associated with the surgical procedure on the display based on the third input;
   optionally wherein the controller is operable to display the steps associated with the surgical procedure as interactable widgets on the display;
   optionally wherein the controller is operable to:

   receive the first input via a first interactable widget on the display; and
   receive the second input via a second interactable widget on the display.

3. The surgical system of Claim 1 or Claim 2, wherein the controller is further operable to:

   receive a user input; and
   adjust the first motor speed to a third motor speed different than the first and second motor speeds based on the user input.

4. A surgical system, comprising:

   an evacuation module that includes a motor and a pump drivable by the motor to draw smoke from a patient; and
   a controller operable to:

   receive an input from a surgical instrument;
   determine a type of tissue based on the input; and
   set a motor speed of the motor based on the type of tissue determined.

5. The surgical system of Claim 4, wherein the input comprises an impedance measurement.

6. The surgical system of Claim 4 or Claim 5, wherein the controller is operable to:

   set a first motor speed of the motor based on a first type of tissue determined; and
   set a second motor speed of the motor different than the first motor speed based on a second type of tissue determined.

7. The surgical system of any one of Claims 4-6, wherein the controller is further operable to determine an amount of time to energize the motor based on the type of tissue determined.

8. The surgical system of any one of Claims 4-7, wherein the motor speed is a first motor speed and the controller is further operable to:

   receive a user input; and
   adjust the first motor speed to a second motor speed different than the first motor speed based on the user input.

9. A surgical system, comprising:

   an evacuation module that includes a motor and a pump drivable by the motor to draw smoke from a patient; and
   a controller operable to:

   receive a first input indicative of a type of surgical instrument;
   receive a second input indicative of a type of surgical procedure;

receive a third input indicative of an amount of time that the surgical instrument was energized during the type of surgical procedure; and

determine a theoretical amount of smoke produced during the amount of time.

10. The surgical system of Claim 9, wherein the controller is further operable to determine a theoretic rate of smoke generation based on the first and second inputs, the controller being further operable to determine the theoretical amount of smoke produced based on the theoretical rate of smoke generation and the amount of time.

11. The surgical system of Claim 9 or Claim 10, wherein the evacuation module further comprises a filter, and wherein the controller is further operable to determine an amount of life remaining of the filter based on the theoretical amount of smoke produced;

optionally wherein the controller is further operable to:

compare the amount of life remaining of the filter to a threshold; and
perform an action based on the comparison;

optionally wherein either:

the surgical system comprises a display and the action comprises generating an alert on the display; or
the action comprises either allowing the motor to be energized for a subsequent surgical procedure based on the amount of life remaining being at or above the threshold, or preventing the motor from being energized for a subsequent surgical procedure based on the amount of life remaining being less than the threshold.

12. The surgical system of any one of Claims 9-11, wherein the type of surgical procedure is selected from the group consisting of an open surgical procedure and a laparoscopic surgical procedure.

13. A computer-readable medium comprising instructions which, when executed by a computer, cause the computer to:

receive a first input indicative of a first step of a surgical procedure;
set, based on the first input, a first motor speed of a motor included in an evacuation module, wherein the evacuation module further includes a pump drivable by the motor to draw smoke from a patient;

receive a second input indicative of a second step of the surgical procedure; and
adjust the first motor speed to a second motor speed different than the first motor speed based on the second input.

14. A computer-readable medium comprising instructions which, when executed by a computer, cause the computer to:

receive an input from a surgical instrument;
determine a type of tissue based on the input; and
set, based on the type of tissue determined, a motor speed of a motor included in an evacuation module, wherein the evacuation module further includes a pump drivable by the motor to draw smoke from a patient.

15. A computer-readable medium comprising instructions which, when executed by a computer, cause the computer to:

receive a first input indicative of a type of surgical instrument;
receive a second input indicative of a type of surgical procedure;
receive a third input indicative of an amount of time that the surgical instrument was energized during the type of surgical procedure; and
determine a theoretical amount of smoke produced during the amount of time.

FIG. 1

FIG. 2

FIG. 3

FIG. 4B

FIG. 4A

FIG. 5

FIG. 6

FIG. 7

EP 4 745 986 A2

FIG. 8

EP 4 745 986 A2

FIG. 9

EP 4 745 986 A2

1000 →

```
┌─────────────────────┐
│   Receive a first    │─── 1002
│   input indicative   │
│   of a type of       │
│   surgical           │
│   procedure          │
└─────────────────────┘
          │
          ▼
┌ ─ ─ ─ ─ ─ ─ ─ ─ ─ ─ ┐
    Display steps       ─── 1004
│   associated with    │
    surgical procedure
│   on a display       │
└ ─ ─ ─ ─ ─ ─ ─ ─ ─ ─ ┘
          │
          ▼
┌─────────────────────┐
│   Receive a second   │─── 1006
│   input indicative   │
│   of a first step    │
│   of the surgical    │
│   procedure          │
└─────────────────────┘
          │
          ▼
┌─────────────────────┐
│   Set a first motor  │─── 1008
│   speed of an        │
│   evacuation motor   │
└─────────────────────┘
          │
          ▼
┌─────────────────────┐
│   Receive a third    │─── 1010
│   input indicative   │
│   of a second step   │
│   of the surgical    │
│   procedure          │
└─────────────────────┘
          │
          ▼
┌─────────────────────┐
│  Adjust the first    │─── 1012
│  motor speed to a    │
│  second motor speed  │
└─────────────────────┘
```

FIG. 10

1100

```
                          ┌─────────────────┐      ─ 1102
                          │   Receive an input │
                          │  from a surgical   │
                          │     device         │
                          └─────────────────┘
                                   │
                                   ▼
                          ┌─────────────────┐      ─ 1104
                          │ Determine a tissue │
                          │      type          │
                          └─────────────────┘
                                   │
                                   ▼
                          ┌─────────────────┐      ─ 1106
                          │  Set a motor speed │
                          │  on an evacuation  │
                          │      motor         │
                          └─────────────────┘
                                   │
                                   ▼
                          ┌ ─ ─ ─ ─ ─ ─ ─ ─ ┐      ─ 1108
                          │  Determine an      │
                          │  amount of time to │
                          │   energize the     │
                          │  evacuation motor  │
                          └ ─ ─ ─ ─ ─ ─ ─ ─ ┘
                                   │
                                   ▼
                          ┌ ─ ─ ─ ─ ─ ─ ─ ─ ┐      ─ 1110
                          │   Energize the     │
                          │  evacuation motor at │
                          │   the motor speed  │
                          └ ─ ─ ─ ─ ─ ─ ─ ─ ┘
                                   │
                                   ▼
                          ┌ ─ ─ ─ ─ ─ ─ ─ ─ ┐      ─ 1112
                          │  De-energize the   │
                          │  evacuation motor  │
                          │  after the amount of │
                          │  time has elapsed  │
                          └ ─ ─ ─ ─ ─ ─ ─ ─ ┘
```

FIG. 11

1200

1202 — Receive a first input indicative of a type of surgical instrument

1204 — Receive a second input indicative of a type of surgical procedure

1206 — Determine a theoretical rate of smoke generation for the surgical instrument during the type of surgical procedure

1208 — Receive a third input indicative of an amount of time the surgical instrument was energized during the type of surgical procedure

1210 — Determine a theoretical amount of smoke generated based on the theoretical rate of smoke generation and the amount of time

1212 — Determine an amount of life remaining of a filter based on the determined theoretical amount of smoke generated

1214 — Remaining filter life > Threshold?

Yes → 1216 — Allow surgical instrument to be energized

No → 1220 — Generate alert

1218 — Prevent surgical instrument from being energized

FIG. 12

FIG. 13

—1400

—1402

Receive an input
indicative of a final
evacuation request

—1404

Determine an initial
volume of
insufflation ($V_I$)

—1406

Determine initial
pressure of
insufflation ($P_I$)

—1408

Determine final
pressure of
insufflation ($P_F$)

—1410

Calculate final
volume of
insufflation ($V_F$)

—1412

Evacuate the final
volume of insufflation
from patient's body
cavity using an
evacuator module

FIG. 14

1500

1502

Receive an input
indicative of a
parameter

1504

No

Parameter >
Threshold?

Yes

1506

Adjust an insufflation
module and/or an
evacuation module

1508

Parameter <
Threshold?

Yes

No

1510

Generate alert

1512

De-energize surgical
instrument

FIG. 15

**EP 4 745 986 A2**

REFERENCES CITED IN THE DESCRIPTION

**Patent documents cited in the description**

- US 950801
- US 950899
- US 951342
- US 11666368 B
- US 10624691 B
- US 11602393 B
- US 11284963 B
- US 11424027 B
- US 11298148 B
- US 20100036373